# EUROPÄISCHE PATENTSCHRIFT

(11) **EP 1 866 291 B1**
(45) Veröffentlichungstag und Bekanntmachung des Hinweises auf die Patenterteilung: **15.07.2009**
(21) Anmeldenummer: 06723578.8
(22) Anmeldetag: 21.03.2006
(51) Int. Cl.: C07D 245/04, A61P 31/04, C07K 5/06, C07K 5/08, C07K 5/10

(54) **ANTIBAKTERIELLE AMID-MAKROZYKLEN VI**
ANTIBACTERIAL AMIDE MACROCYCLES VI
MACROCYCLES D'AMIDE ANTIBACTERIENS VI

(30) Priorität: 30.03.2005 DE 102005014247
(43) Veröffentlichungstag der Anmeldung: 19.12.2007
(73) Patentinhaber: AiCuris GmbH & Co. KG, 42117 Wuppertal (DE)
(72) Erfinder: ENDERMANN, Rainer, 42113 Wuppertal (DE); EHLERT, Kerstin, 42553 Velbert (DE); RADDATZ, Siegfried, 51065 Köln (DE); MICHELS, Martin, West Haven, Connecticut 06516-4140 (US); CANCHO-GRANDE, Yolanda, 51373 Leverkusen (DE); WEIGAND, Stefan, 82377 Penzberg (DE); FISCHER, Karin, 42699 Solingen (DE)
(74) Vertreter: Weller, Wolfgang
(86) Internationale Anmeldenummer: PCT/EP2006/002564
(87) Internationale Veröffentlichungsnummer: WO 2006/103009

(56) Entgegenhaltungen:
- DE-A1- 10 226 921

## Beschreibung

Die Erfindung betrifft antibakterielle Amid-Makrozyklen und Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von bakteriellen Infektionen.

In WO 03/106480, WO 04/012816, WO 05/033129, WO 05/058943, WO 05/100380 und WO 05/118613 werden antibakteriell wirkende Makrozyklen vom Biphenomycin B Typ mit Amid- bzw. Estersubstituenten beschrieben.

In US 3,452,136, Dissertation R. U. Meyer, Universität Stuttgart, Deutschland 1991, Dissertation V. Leitenberger, Universität Stuttgart, Deutschland 1991, Synthesis (1992), (10), 1025-30, J. Chem. Soc., Perkin Trans. 1 (1992), (1), 123-30, J. Chem. Soc., Chem. Commun. (1991), (10), 744, Synthesis (1991), (5), 409-13, J. Chem. Soc., Chem. Commun. (1991), (5), 275-7, J. Antibiot. (1985), 38(11), 1462-8, J. Antibiot. (1985), 38(11), 1453-61, wird der Naturstoff Biphenomycin B als antibakteriell wirksam beschrieben. Teilschritte der Synthese von Biphenomycin B werden in Synlett (2003), 4, 522-526 beschrieben.

Chirality (1995), 7(4), 181-92, J. Antibiot. (1991), 44(6), 674-7, J. Am. Chem. Soc. (1989), 111(19), 7323-7, J. Am. Chem. Soc. (1989), 111(19), 7328-33, J. Org. Chem. (1987), 52(24), 5435-7, Anal. Biochem. (1987), 165(1), 108-13, J. Org. Chem. (1985), 50(8), 1341-2, J. Antibiot. (1993), 46(3), C-2, J. Antibiot. (1993), 46(1), 135-40, Synthesis (1992), (12), 1248-54, Appl. Environ. Microbiol. (1992), 58(12), 3879-8, J. Chem. Soc., Chem. Commun. (1992), (13), 951-3 beschreiben einen strukturell verwandten Naturstoff, Biphenomycin A, der am Makrozyklus eine weitere Substitution mit einer Hydroxygruppe aufweist.

Die Naturstoffe entsprechen hinsichtlich ihrer Eigenschaften nicht den Anforderungen, die an antibakterielle Arzneimittel gestellt werden. Auf dem Markt sind zwar strukturell andersartige antibakteriell wirkende Mittel vorhanden, es kann aber regelmäßig zu einer Resistenzentwicklung kommen. Neue Mittel für eine gute und wirksamere Therapie sind daher wünschenswert.

Eine Aufgabe der vorliegenden Erfindung ist es daher, neue und alternative Verbindungen mit gleicher oder verbesserter antibakterieller Wirkung zur Behandlung von bakteriellen Erkrankungen bei Menschen und Tieren zur Verfügung zu stellen.

Überraschenderweise wurde gefunden, dass bestimmte Derivate dieser Naturstoffe, worin die Carboxylgruppe des Naturstoffs gegen eine tertiäre Amidgruppe ausgetauscht wird, die eine basische Gruppe enthält, gegen Biphenomycin resistente *S. aureus* Stämme (RN4220Bi^{R} und T17) antibakteriell wirksam sind.

Weiterhin zeigen die Derivate gegen *S*. *aureus* Wildtyp-Stämme und Biphenomycin resistente *S*. *aureus* Stämme eine verbesserte Spontanresistenz-Frequenz.

Gegenstand der Erfindung sind Verbindungen der Formel 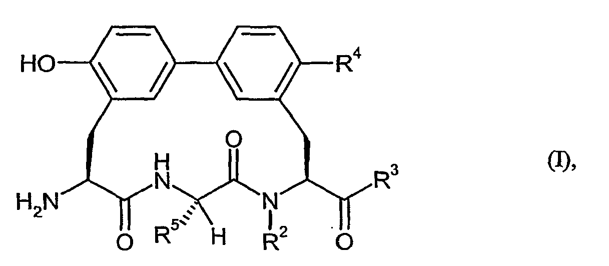 bei denen
- R⁵: gleich eine Gruppe der Formel 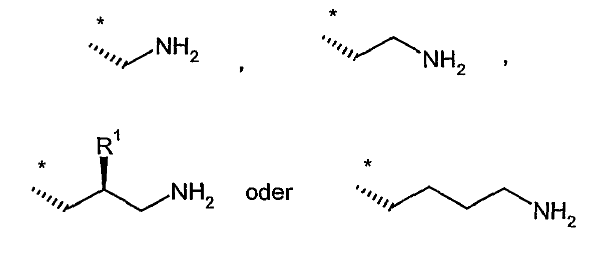 ist,
wobei
* die Anknüpfstelle an das Kohlenstoffatom ist,
R¹ gleich Wasserstoff oder Hydroxy ist,
- R²: gleich Wasserstoff, Methyl oder Ethyl ist,
- R³: gleich eine Gruppe der Formel 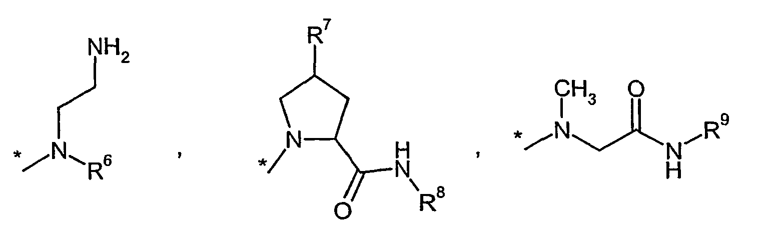 oder 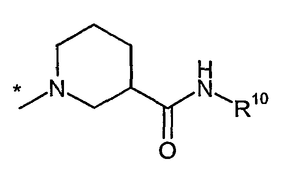 ist,
wobei
- *: die Anknüpfstelle an das Stickstoffatom ist,
- R⁶: gleich eine Gruppe der Formel 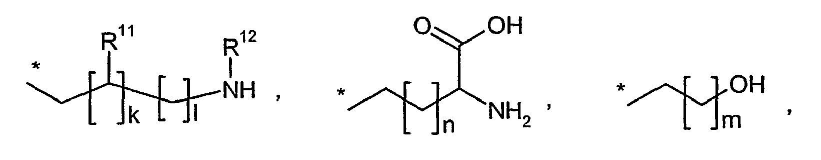 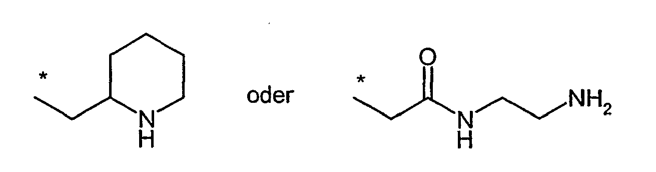 ist,
worin
* die Anknüpfstelle an das Stickstoffatom ist,
R¹¹ gleich Wasserstoff, Amino oder Hydroxy ist,
R¹² gleich Wasserstoff oder Methyl ist,
k eine Zahl 0 oder 1 ist,
l eine Zahl 1, 2, 3 oder 4 ist,
und
m und n unabhängig voneinander eine Zahl 1, 2 oder 3 sind,
- R⁷: gleich Wasserstoff, Amino oder Hydroxy ist,
- R⁸, R⁹ und R¹⁰: unabhängig voneinander eine Gruppe der Formel 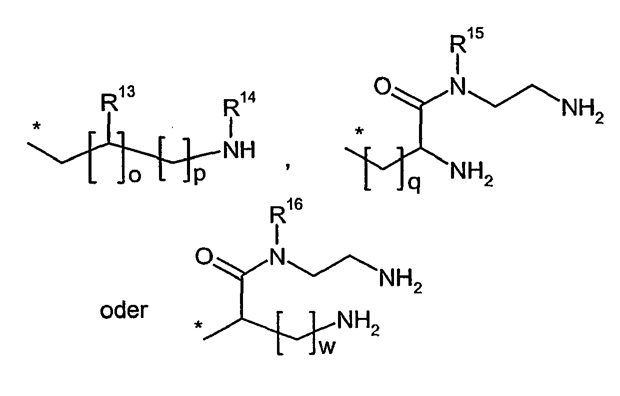 sind,
worin
* die Anknüpfstelle an das Stickstoffatom ist,
R¹³ gleich Wasserstoff, Amino oder Hydroxy ist,
R¹⁴ gleich Wasserstoff oder Methyl ist,
R¹⁵ und R¹⁶ unabhängig voneinander Wasserstoff, Aminoethyl oder Hydroxyethyl sind,
o eine Zahl 0 oder 1 ist,
p, q und w unabhängig voneinander eine Zahl 1, 2, 3 oder 4 sind,
- R⁴: gleich Wasserstoff, Hydroxy, Halogen, Amino oder Methyl ist,
und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich durch bekannte Verfahren wie Chromatographie an chiraler Phase oder Kristallisation mit chiralen Aminen oder chiralen Säuren die stereoisomer einheitlichen Bestandteile in bekannter Weise isolieren.

Die Erfindung betrifft in Abhängigkeit von der Struktur der Verbindungen auch Tautomere der Verbindungen.

Als Salze sind im Rahmen der Erfmdung physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure, Trifluoressigsäure und Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Dihydroabietylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Halogen steht für Fluor, Chlor, Brom und Jod. Ein Symbol # an einem Kohlenstoffatom bedeutet, dass die Verbindung hinsichtlich der Konfiguration an diesem Kohlenstoffatom in enantiomerenreiner Form vorliegt, worunter im Rahmen der vorliegenden Erfindung ein Enantiomerenüberschuss (enantiomeric excess) von mehr als 90 % verstanden wird (> 90% ee).

In den Formeln der Gruppen, die für R³ stehen, steht der Endpunkt der Linie, neben der jeweils ein * steht, nicht für ein Kohlenstoffatom beziehungsweise eine CH₂-Gruppe sondern ist Bestandteil der Bindung zu der Carbonyl-Gruppe, an das R³ gebunden ist.

In den Formeln der Gruppen, für die R⁵ stehen kann, steht der Endpunkt der Linie, neben der jeweils ein * steht, nicht für ein Kohlenstoffatom beziehungsweise eine CH₂-Gruppe sondern ist Bestandteil der Bindung zu dem Kohlenstoffatom, an das R⁷ gebunden ist.

In den Formeln der Gruppen, die für R⁶, R⁸, R⁹ und R¹⁰ stehen, steht der Endpunkt der Linie, neben der jeweils ein * steht, nicht für ein Kohlenstoffatom beziehungsweise eine CH₂-Gruppe sondern ist Bestandteil der Bindung zu dem Stickstoffatom, an das R⁶, R⁸, R⁹ und R¹⁰ gebunden sind.

Bevorzugt im Rahmen der vorliegenden Erfindung sind auch Verbindungen der Formel 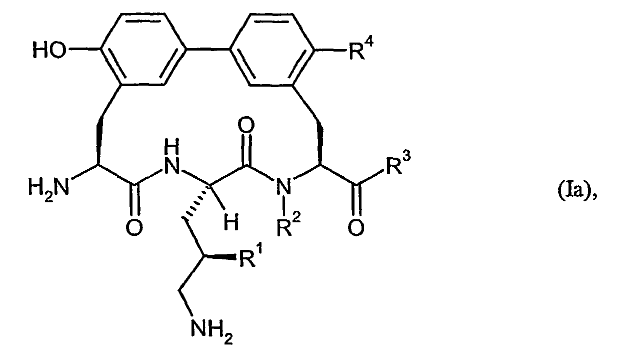 bei denen
- R¹: gleich Wasserstoff oder Hydroxy ist,
- R²: gleich Wasserstoff, Methyl oder Ethyl ist,
- R³: wie oben definiert ist,
- R⁴: gleich Wasserstoff, Hydroxy, Halogen, Amino oder Methyl ist,
und ihre Salze, ihre Solvate und die Solvate ihrer Salze.

Bevorzugt im Rahmen der vorliegenden Erfindung sind auch Verbindungen der Formel (I) oder (Ia), bei denen
- R²: gleich Wasserstoff ist.

Bevorzugt im Rahmen der vorliegenden Erfindung sind auch Verbindungen der Formel (I) oder (Ia), bei denen
- R⁴: gleich Wasserstoff, Hydroxy, Chlor oder Methyl ist.

Bevorzugt im Rahmen der vorliegenden Erfindung sind auch Verbindungen der Formel (I) oder (Ia), bei denen
- R⁴: gleich Hydroxy ist.

Bevorzugt im Rahmen der vorliegenden Erfindung sind auch Verbindungen der Formel (I) oder (Ia), bei denen
- R⁵: gleich eine Gruppe der Formel 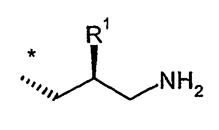 ist,
wobei
* die Anknüpfstelle an das Kohlenstoffatom ist,
R¹ gleich Wasserstoff oder Hydroxy ist.

Bevorzugt im Rahmen der vorliegenden Erfindung sind auch Verbindungen der Formel (I) oder (Ia), bei denen
- R⁵: gleich eine Gruppe der Formel 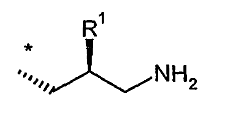 ist,
wobei
* die Anknüpfstelle an das Kohlenstoffatom ist,
R¹ gleich Wasserstoff oder Hydroxy ist,
- R²: gleich Wasserstoff ist,
- R³: gleich eine Gruppe der Formel 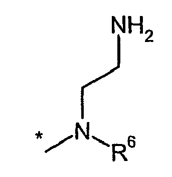 ist,
wobei
* die Anknüpfstelle an das Stickstoffatom ist,
R⁶ gleich eine Gruppe der Formel 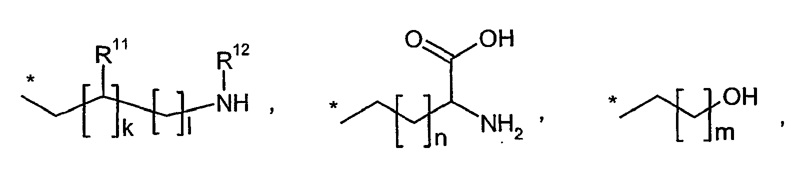 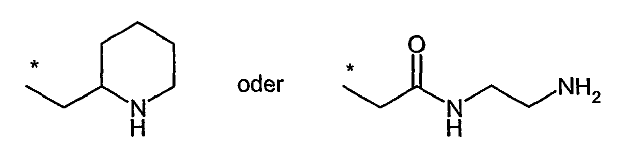 ist,
worin
* die Anknüpfstelle an das Stickstoffatom ist,
R¹¹ gleich Wasserstoff, Amino oder Hydroxy ist,
R¹² gleich Wasserstoff oder Methyl ist,
k eine Zahl 0 oder 1 ist,
l eine Zahl 1, 2, 3 oder 4 ist,
und
m und n unabhängig voneinander eine Zahl 1, 2 oder 3 sind,
- R⁴: gleich Hydroxy ist,
und ihre Salze, ihre Solvate und die Solvate ihrer Salze.

Bevorzugt im Rahmen der vorliegenden Erfindung sind auch Verbindungen der Formel (I) oder (Ia), bei denen
- R⁵: gleich eine Gruppe der Formel 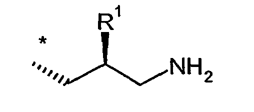 ist,
wobei
* die Anknüpfstelle an das Kohlenstoffatom ist,
R¹ gleich Wasserstoff oder Hydroxy ist,
- R²: gleich Wasserstoff ist,
- R³: gleich eine Gruppe der Formel 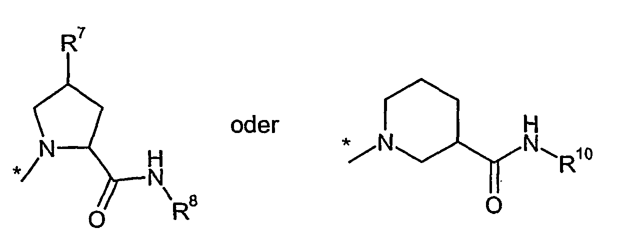 ist,
wobei
* die Anknüpfstelle an das Stickstoffatom ist,
R⁷ gleich Wasserstoff, Amino oder Hydroxy ist,
R⁸ und R¹⁰ unabhängig voneinander eine Gruppe der Formel 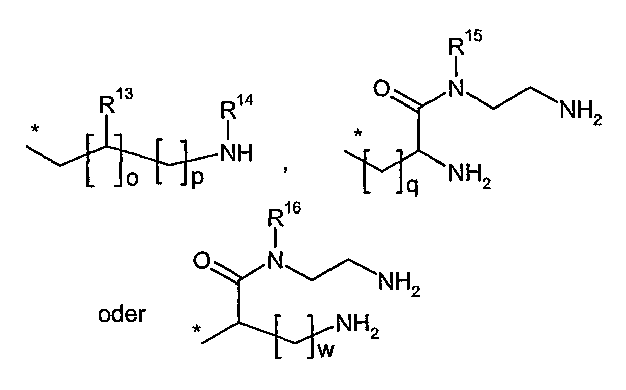 sind,
worin
* die Anknüpfstelle an das Stickstoffatom ist,
R¹³ gleich Wasserstoff, Amino oder Hydroxy ist,
R¹⁴ gleich Wasserstoff oder Methyl ist,
R¹⁵ und R¹⁶ unabhängig voneinander Wasserstoff, Aminoethyl oder Hydroxyethyl sind,
o eine Zahl 0 oder 1 ist,
p, q und w unabhängig voneinander eine Zahl 1, 2, 3 oder 4 sind,
- R⁴: gleich Hydroxy ist,
und ihre Salze, ihre Solvate und die Solvate ihrer Salze.

Bevorzugt im Rahmen der vorliegenden Erfindung sind auch Verbindungen der Formel (I) oder (Ia), bei denen
- R⁵: gleich eine Gruppe der Formel 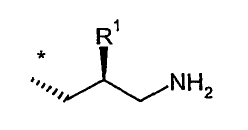 ist,
wobei
* die Anknüpfstelle an das Kohlenstoffatom ist,
R¹ gleich Wasserstoff oder Hydroxy ist,
- R²: gleich Wasserstoff ist,
- R³: gleich eine Gruppe der Formel 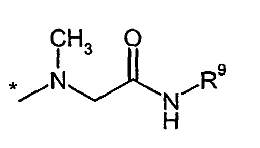 ist,
wobei
* die Anknüpfstelle an das Stickstoffatom ist,
R⁹ eine Gruppe der Formel 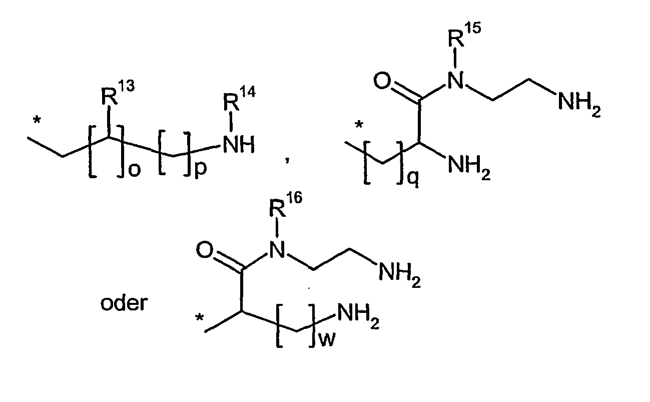 ist,
worin
* die Anknüpfstelle an das Stickstoffatom ist,
R¹³ gleich Wasserstoff, Amino oder Hydroxy ist,
R¹⁴ gleich Wasserstoff oder Methyl ist,
R¹⁵ und R¹⁶ unabhängig voneinander Wasserstoff, Aminoethyl oder Hydroxyethyl sind,
o eine Zahl 0 oder 1 ist,
p, q und w unabhängig voneinander eine Zahl 1, 2, 3 oder 4 sind,
- R⁴: gleich Hydroxy ist,
und ihre Salze, ihre Solvate und die Solvate ihrer Salze.

Gegenstand der Erfindung ist weiterhin ein Verfahren zur Herstellung der Verbindungen der Formel (I) oder ihrer Salze, ihrer Solvate oder der Solvate ihrer Salze, wobei nach Verfahren
[A] Verbindungen der Formel 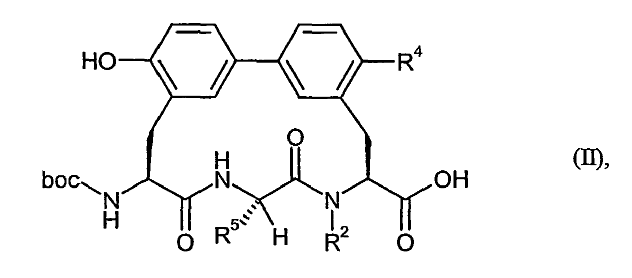 worin R², R⁴ und R⁵ die oben angegebene Bedeutung haben und boc gleich tert-Butoxycarbonyl ist,
   in einem zweistufigen Verfahren zunächst in Gegenwart von einem oder mehreren Dehydratisierungsreagenzien mit Verbindungen der Formel

   HR³ (III),

   worin R³ die oben angegebene Bedeutung hat,
   und anschließend mit einer Säure und/oder durch Hydrogenolyse umgesetzt werden,
   oder
[B] Verbindungen der Formel 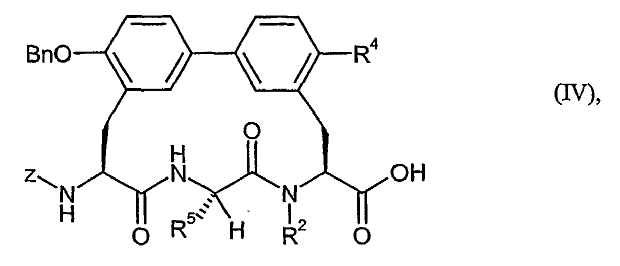 worin R², R⁴ und R⁵ die oben angegebene Bedeutung haben und Z gleich Benzyloxycarbonyl ist,
   in einem zweistufigen Verfahren zunächst in Gegenwart von einem oder mehreren Dehydratisierungsreagenzien mit Verbindungen der Formel

   HR³ (III),

   worin R³ die oben angegebene Bedeutung hat,
   und anschließend mit einer Säure oder durch Hydrogenolyse umgesetzt werden.

Die freie Base der Salze kann zum Beispiel durch Chromatographie an einer Reversed Phase Säule mit einem Acetonitril-Wasser-Gradienten unter Zusatz einer Base erhalten werden, insbesondere durch Verwendung einer RP18 Phenomenex Luna C18(2) Säule und Diethylamin als Base.

Weiterer Gegenstand der Erfindung ist ein Verfahren zur Herstellung der Verbindungen der Formel (I) oder ihrer Solvate nach Anspruch 1, bei dem Salze der Verbindungen oder Solvate der Salze der Verbindungen durch Chromatographie unter Zusatz einer Base in die Verbindungen überführt werden.

Die Hydroxygruppe an R¹ ist gegebenenfalls während der Umsetzung mit Verbindungen der Formel (III) mit einer *tert*-Butyldimethylsilyl-Gruppe geschützt, die im zweiten Reaktionsschritt abgespalten wird.

Reaktive Funktionalitäten in dem Rest R³ von Verbindungen der Formel (III) werden bereits geschützt mit in die Synthese eingebracht, bevorzugt sind säurelabile Schutzgruppen (z.B. boc). Nach erfolgter Umsetzung zu Verbindungen der Formel (I) können die Schutzgruppen durch Entschützungsreaktion abgespalten werden. Dies geschieht nach Standardverfahren der Schutzgruppenchemie. Bevorzugt sind Entschützungsreaktionen unter sauren Bedingungen oder durch Hydrogenolyse.

Die Umsetzung der ersten Stufe der Verfahren [A] und [B] erfolgt im Allgemeinen in inerten Lösungsmitteln, gegebenenfalls in Gegenwart einer Base, bevorzugt in einem Temperaturbereich von 0°C bis 40°C bei Normaldruck.

Als Dehydratisierungsreagenzien eignen sich hierbei beispielsweise Carbodiimide wie z.B. *N,N'-*Diethyl-, *N,N'*-Dipropyl-, *N,N'*-Diisopropyl-, *N,N'*-Dicyclohexylcarbodiimid, *N*-(3-Dimethylamino-isopropyl)-*N'*-ethylcarbodiimid-Hydrochlorid (EDC), *N*-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-*N,N,N',N'*-tetra-methyluroniumhexafluorophosphat (HBTU), 2-(2-Oxo-1-(2H)-pyridyl)-1,1,3,3-tetramethyluroniumtetrafluoroborat (TPTU) oder O-(7-Azabenzotriazol-1-yl)-*N,N,N',N'*-tetramethyluroniumhexafluorophosphat (HATU), oder 1-Hydroxybenztriazol (HOBt), oder Benzotriazol-1-yloxytris(dimethylamino)-phosphoniumhexafluoro-phosphat (BOP), oder Benzotriazol-1-yloxytris(pyrrolidino)-phosphoniumhexafluorophosphat (PyBOP), oder Mischungen aus diesen, oder Mischung aus diesen zusammen mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Vorzugsweise wird die Kondensation mit HATU in Gegenwart einer Base, insbesondere Diisopropylethylamin, oder mit PyBOP in Gegenwart einer Base, insbesondere Diisopropylethylamin, durchgeführt.

Inerte Lösungsmittel sind beispielsweise Halogenkohlenwasserstoffe wie Dichlormethan oder Trichlormethan, Kohlenwasserstoff wie Benzol, oder Nitromethan, Dioxan, Dimethylformamid oder Acetonitril. Ebenso ist es möglich, Gemische der Lösemittel einzusetzen. Besonders bevorzugt ist Dimethylformamid.

Die Umsetzung mit einer Säure in der zweiten Stufe der Verfahren [A] und [B] erfolgt bevorzugt in einem Temperaturbereich von 0°C bis 40°C bei Normaldruck.

Als Säuren eignen sich hierbei Chlorwasserstoff in Dioxan, Bromwasserstoff in Essigsäure oder Trifluoressigsäure in Methylenchlorid.

Die Hydrogenolyse in der zweiten Stufe des Verfahrens [B] erfolgt im Allgemeinen in einem Lösungsmittel in Gegenwart von Wasserstoff und Palladium auf Aktivkohle, bevorzugt in einem Temperaturbereich von 0°C bis 40°C bei Normaldruck.

Lösungsmittel sind beispielsweise Alkohole wie Methanol, Ethanol, n-Propanol oder iso-Propanol, in einem Gemisch mit Wasser und Eisessig, bevorzugt ist ein Gemisch aus Ethanol, Wasser und Eisessig.

Die Verbindungen der Formel (III) sind bekannt oder können analog bekannten Verfahren hergestellt werden.

Die Verbindungen der Formel (II) sind bekannt oder können hergestellt werden, indem Verbindungen der Formel 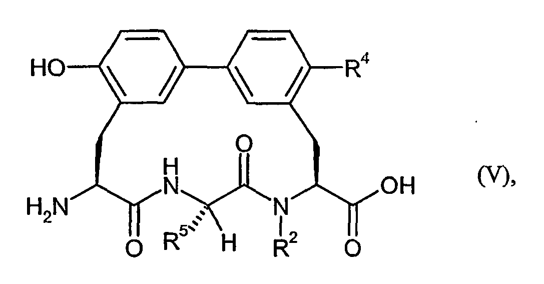 worin R², R⁴ und R⁵ die oben angegebene Bedeutung haben,
mit Di-(*tert*-butyl)-dicarbonat in Gegenwart einer Base umgesetzt werden.

Die Umsetzung erfolgt im Allgemeinen in einem Lösungsmitteln, bevorzugt in einem Temperaturbereich von 0°C bis 40°C bei Normaldruck.

Basen sind beispielsweise Alkalihydroxide wie Natrium- oder Kaliumhydroxid, oder Alkalicarbonate wie Cäsiumcarbonat, Natrium- oder Kaliumcarbonat, oder andere Basen wie DBU, Triethylamin oder Diisopropylethylamin, bevorzugt ist Natriumhydroxid oder Natriumcarbonat.

Lösungsmittel sind beispielsweise Halogenkohlenwasserstoffe wie Methylenchlorid oder 1,2-Dichlorethan, Alkohole wie Methanol, Ethanol oder iso-Propanol, oder Wasser.

Vorzugsweise wird die Umsetzung mit Natriumhydroxid in Wasser oder Natriumcarbonat in Methanol durchgeführt.

Die Verbindungen der Formel (V) sind bekannt oder können hergestellt werden, indem Verbindungen der Formel 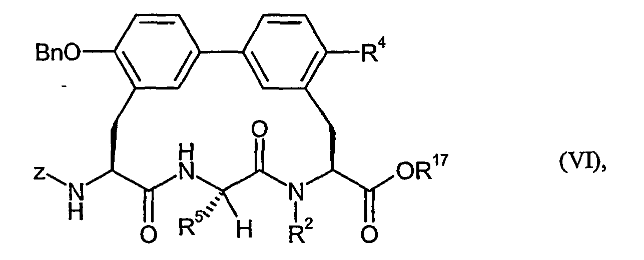 worin R², R⁴ und R⁵ die oben angegebene Bedeutung haben, und
- R¹⁷: gleich Benzyl, Methyl oder Ethyl ist,
mit einer Säure oder durch Hydrogenolyse, wie für die zweite Stufe des Verfahrens [B] beschrieben, gegebenenfalls durch anschließende Umsetzung mit einer Base zur Verseifung des Methyl- oder Ethylesters, umgesetzt werden.

Die Verseifung kann zum Beispiel erfolgen, wie bei der Umsetzung von Verbindungen der Formel (VI) zu Verbindungen der Formel (IV) beschrieben.

Die Verbindungen der Formel (IV) sind bekannt oder können hergestellt werden, indem in Verbindungen der Formel (VI) der Benzyl-, Methyl- oder Ethylester verseift wird.

Die Umsetzung erfolgt im Allgemeinen in einem Lösungsmitteln, in Gegenwart einer Base, bevorzugt in einem Temperaturbereich von 0°C bis 40°C bei Normaldruck.

Basen sind beispielsweise Alkalihydroxide wie Lithium-, Natrium- oder Kaliumhydroxid, bevorzugt ist Lithiumhydroxid.

Lösungsmittel sind beispielsweise Halogenkohlenwasserstoffe wie Dichlormethan oder Trichlormethan, Ether wie Tetrahydrofuran oder Dioxan, oder Alkohole wie Methanol, Ethanol oder Iso-propanol, oder Dimethylformamid. Ebenso ist es möglich, Gemische der Lösungsmittel oder Gemische der Lösungsmittel mit Wasser einzusetzen. Besonders bevorzugt sind Tetrahydrofuran oder ein Gemisch aus Methanol und Wasser.

Die Verbindungen der Formel (VI) sind bekannt oder können hergestellt werden, indem Verbindungen der Formel 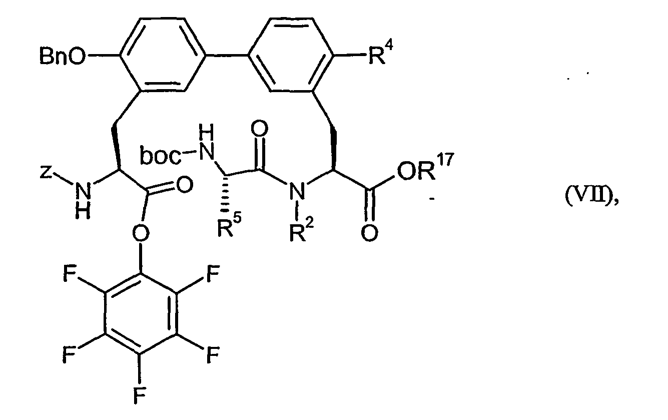 worin R², R⁴, R⁵ und R¹⁷ die oben angegebene Bedeutung haben,
in der ersten Stufe mit Säuren, wie für die zweite Stufe der Verfahren [A] und [B] beschrieben, und in der zweiten Stufe mit Basen umgesetzt werden.

In der zweiten Stufe erfolgt die Umsetzung mit Basen im Allgemeinen in einem Lösungsmitteln, bevorzugt in einem Temperaturbereich von 0°C bis 40°C bei Normaldruck.

Basen sind beispielsweise Alkalihydroxide wie Natrium- oder Kaliumhydroxid, oder Alkalicarbonate wie Cäsiumcarbonat, Natrium- oder Kaliumcarbonat, oder andere Basen wie DBU, Triethylamin oder Diisopropylethylamin, bevorzugt ist Triethylamin.

Lösungsmittel sind beispielsweise Halogenkohlenwasserstoffe wie Chloroform, Methylenchlorid oder 1,2-Dichlorethan, oder Tetrahydrofuran, oder Gemische der Lösungsmittel, bevorzugt ist Methylenchlorid oder Tetrahydrofuran.

Die Verbindungen der Formel (VII) sind bekannt oder können hergestellt werden, indem Verbindungen der Formel 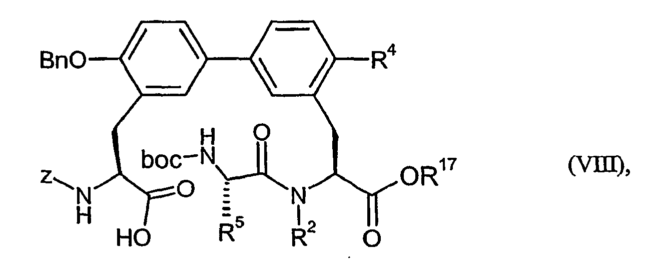 worin R², R⁴, R⁵ und R¹⁷ die oben angegebene Bedeutung haben,
mit Pentafluorphenol in Gegenwart von Dehydratisierungsreagenzien, wie für die erste Stufe der Verfahren [A] und [B] beschrieben, umgesetzt werden.

Die Umsetzung erfolgt bevorzugt mit DMAP und EDC in Dichlormethan in einem Temperaturbereich von -40°C bis 40°C bei Normaldruck.

Die Verbindungen der Formel (VIII) sind bekannt oder können hergestellt werden, indem Verbindungen der Formel 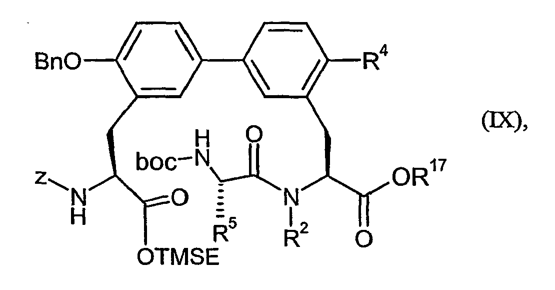 worin R², R⁴, R⁵ und R¹⁷ die oben angegebene Bedeutung haben,
mit Fluorid, insbesondere mit Tetrabutylammoniumfluorid, umgesetzt werden.

Die Umsetzung erfolgt im Allgemeinen in einem Lösungsmitteln, bevorzugt in einem Temperaturbereich von -10°C bis 30°C bei Normaldruck.

Inerte Lösungsmittel sind beispielsweise Halogenkohlenwasserstoffe wie Dichlormethan, oder Kohlenwasserstoffe wie Benzol oder Toluol, oder Ether wie Tetrahydrofuran oder Dioxan, oder Dimethylformamid. Ebenso ist es möglich, Gemische der Lösemittel einzusetzen. Bevorzugte Lösungsmittel sind Tetrahydrofuran und Dimethylformamid.

Die Verbindungen der Formel (IX) sind bekannt oder können hergestellt werden, indem Verbindungen der Formel 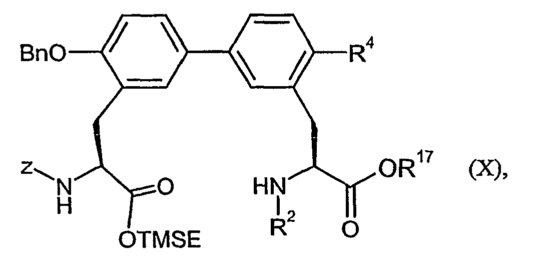 worin R², R⁴ und R¹⁷ die oben angegebene Bedeutung haben,
mit Verbindungen der Formel 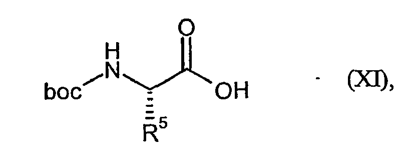 worin R⁵ die oben angegebene Bedeutung hat,
in Gegenwart von Dehydratisierungsreagenzien, wie für die erste Stufe der Verfahren [A] und [B] beschrieben, umgesetzt werden.

Die Verbindungen der Formel (X) sind bekannt oder können analog den im Beispielteil beschriebenen Verfahren hergestellt werden.

Die Verbindungen der Formel (XI) sind bekannt oder können analog bekannten Verfahren hergestellt werden.

Die erfindungsgemäßen Verbindungen zeigen ein nicht vorhersehbares, wertvolles pharmakologisches und pharmakokinetisches Wirkspektrum.

Sie eignen sich daher zur Verwendung als Arzneimittel zur Behandlung und/oder Prophylaxe von Krankheiten bei Menschen und Tieren.

Die erfindungsgemäßen Verbindungen können aufgrund ihrer pharmakologischen Eigenschaften allein oder in Kombination mit anderen Wirkstoffen zur Behandlung und/oder Prophylaxe von Infektionskrankheiten, insbesondere von bakteriellen Infektionen, eingesetzt werden.

Beispielsweise können lokale und/oder systemische Erkrankungen behandelt und/oder verhindert werden, die durch die folgenden Erreger oder durch Mischungen der folgenden Erreger verursacht werden:
Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytocy), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft aufzufassen. Als Krankheiten, die durch die genannten Erreger oder Mischinfekionen verursacht und durch die erfindungsgemäßen topisch anwendbaren Zubereitungen verhindert, gebessert oder geheilt werden können, seien beispielsweise genannt:
Infektionskrankheiten beim Menschen wie z. B. septische Infektionen, Knochen- und Gelenkinfektionen, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, septische Arthritis, Mastitis, Tonsillitis, Genital-Infektionen und Augeninfektionen.

Außer beim Menschen können bakterielle Infektionen auch bei anderen Spezies behandelt werden. Beispielhaft seien genannt:
Schwein: Coli-diarrhoe, Enterotoxamie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Hamwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie z.B. Pasteurella, Brucella, Campylobacter, Listeria, Erysipelothris, Corynebakterien, Borellia, Treponema, Nocardia, Rikettsie, Yersinia, erweitert. Weiterer Gegenstand der vorliegenden Erfindung ist der Einsatz der erfindungsgemäßen Verbindungen zur Behandlung und/oder Prophylaxe von Erkrankungen, vorzugsweise von bakteriellen Krankheiten, insbesondere von bakteriellen Infektionen.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer antibakteriell wirksamen Menge der erfindungsgemäßen Verbindungen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfmdungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei parenteraler Applikation Mengen von etwa 5 bis 250 mg/kg Körpergewicht je 24 h zur Erzielung wirksamer Ergebnisse zu verabreichen. Bei oraler Applikation beträgt die Menge etwa 5 bis 100 mg/kg Körpergewicht je 24 h.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Verwendete Abkürzungen:

- abs.: absolut
- aq.: wässrig
- Bn: Benzyl
- boc: *tert*-Butoxycarbonyl
- Bsp.: Beispiel
- CDCl₃: Chloroform
- CH: Cyclohexan
- d: dublett (im ¹H-NMR)
- dd: dublett von dublett (im ¹H-NMR)
- DC: Dünnschichtchromatographie
- DCC: Dicyclohexylcarbodiimid
- DIC: Diisopropylcarbodiimid
- DIEA: Diisopropylethylamin (Hünig-Base)
- DMSO: Dimethylsulfoxid
- DMAP: 4-*N,N*-Dimethylaminopyridin
- DMF: Dimethylformamid
- d. Th.: der Theorie
- EDC: *N'*-(3-Dimethylaminopropyl)-*N*-ethylcarbodiimid x HCl
- EE: Ethylacetat (Essigsäureethylester)
- ESI: Elektrospray-Ionisation (bei MS)
- Fmoc: 9-Fluorenylmethoxycarbonyl
- ges.: gesättigt
- HATU: *O*-(7-Azabenzotriazol-1-yl)-*N,N,N',N'*-tetramethyluroniumhexafluorophosphat
- HBTU: *O*-(Benzotriazol-1-yl)-*N,N,N',N'*-tetramethyluroniumhexafluorophosphat
- HOBt: 1-Hydroxy-1H-benzotriazol x H₂O
- h: Stunde(n)
- HPLC: Hochdruck-, Hochleistungsflüssigchromatographie
- LC-MS: Flüssigchromatographie-gekoppelte Massenspektroskopie
- m: multiplett (im ¹H-NMR)
- min: Minute
- MS: Massenspektroskopie
- NMR: Kernresonanzspektroskopie
- MTBE: Methyl-*tert*-butylether
- Pd/C: Palladium/Kohle
- PFP: Pentafluorphenol
- proz.: Prozent
- PyBOP: Benzotriazol-1-yloxytris(pyrrolidino)-phosphoniumhexafluorophosphat
- q: quartett (im ¹H-NMR)
- R_{f}: Retentionsindex (bei DC)
- RP: Reverse Phase (bei HPLC)
- RT: Raumtemperatur
- Rₜ: Retentionszeit (bei HPLC)
- s: singulett (im ¹H-NMR)
- t: triplett (im ¹H-NMR)
- TBS: *tert*-Butyldimethylsilyl
- TFA: Trifluoressigsäure
- THF: Tetrahydrofuran
- TMSE: 2-(Trimethylsilyl)-ethyl
- TPTU: 2-(2-Oxo-1(2H)-pyridyl)-1,1,3,3-tetramethyluroniumtetrafluoroborat
- Z: Benzyloxycarbonyl

### LC-MS- und HPLC-Methoden:

**Methode 1 (LC-MS):** Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Methode 2 (LC-MS):** Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

**Methode 3 (LC-MS):** Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208- 400 nm.

**Methode 4 (LC-MS):** Gerätetyp MS: Micromass ZQ; Gerätetyp HPLC: Waters Alliance 2795; Säule: Merck Chromolith SpeedROD RP-18e 50 mm x 4.6 mm; Eluent A: Wasser + 500 µl 50%ige Ameisensäure / 1; Eluent B: Acetonitril + 500 µl 50%ige Ameisensäure / 1; Gradient: 0.0 min 10%B → 3.0 min 95%B → 4.0 min 95%B; Ofen: 35°C; Fluss: 0.0 min 1.0 ml/min → 3.0 min 3.0 ml/min → 4.0 min 3.0 ml/min; UV-Detektion: 210 nm.

### Methode 5 (LC-MS): Instrument: Micromass Platform LCZ mit HPLC Agilent Serie 1100; Säule:

Thermo HyPURITY Aquastar 3µ 50 mm x 2.1 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100%A → 0.2 min 100%A → 2.9 min 30%A → 3.1 min 10%A → 5.5 min 10%A; Ofen: 50°C; Fluss: 0.8 ml/min; UV-Detektion: 210 nm.

**Methode 6 (LC-MS):** Instrument: Micromass Platform LCZ mit HPLC Agilent Serie 1100; Säule: Thermo Hypersil GOLD-3µ 20 x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100%A → 0.2 min 100%A → 2.9 min 30%A → 3.1 min 10%A → 5.5 min 10%A; Ofen: 50°C; Fluss: 0.8 ml/min; UV-Detektion: 210 nm.

### Ausgangsverbindungen

### Beispiel 1A

### Benzyl-{(1S)-4-[(tert-butoxycarbonyl)amino]-1-[({2-[(tert-butoxycarbonyl)amino]ethyl}amino)-carbonyl]butyl}carbamat

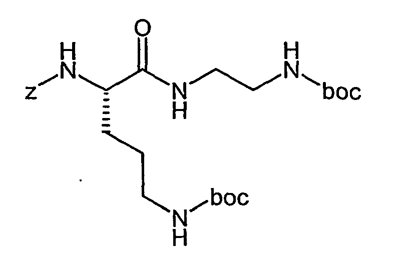

Unter Argon werden 300 mg (0.82 mmol) *N*²-[(Benzyloxy)carbonyl]-*N*⁵-(*tert*-butoxycarbonyl)-*L-*omithin und 171 mg (1.06 mmol) *tert*-Butyl-(2-aminoethyl)carbamat in 6 ml Dimethylformamid gelöst. Bei 0°C (Eisbad) werden dann 204 mg (1.06 mmol) EDC und 33 mg (0.25 mmol) HOBt zugegeben. Es wird langsam auf RT erwärmt und für 12 h bei RT gerührt. Die Lösung wird im Vakuum eingeengt und der Rückstand wird mit Essigsäureethylester aufgenommen. Die organische Phase wird nacheinander mit gesättigter Natriumhydrogencarbonat- und Natriumchlorid-Lösung gewaschen, über Magnesiumsulfat getrocknet und im Vakuum eingedampft. Der verbleibende Feststoff wird im Hochvakuum getrocknet.

Ausbeute: 392 mg (94% d. Th.)

LC-MS (Methode 1): Rₜ = 2.36 min.

MS (ESI): m/z = 509 (M+H)⁺

### Beispiel 2A

### N⁵-(tert-Butoxycarbonyl)-N-{2-[(tert-butoxycarbonyl)amino]ethyl}-L-ornithinamid

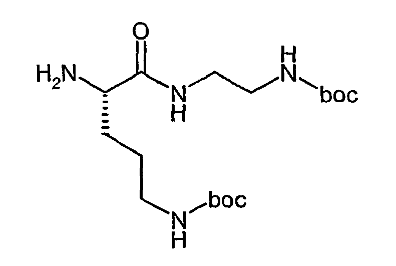

Eine Lösung von 390 mg (0.77 mmol) Benzyl-{(1*S*)-4-[(*tert*-butoxycarbonyl)amino]-1-[({2-[(*tert-*butoxycarbonyl)amino]ethyl}amino)carbonyl]butyl}carbamat (Beispiel 1A) in 50 ml Ethanol wird nach Zugabe von 40 mg Palladium auf Aktivkohle (10%ig) 4 h bei RT und Normaldruck hydriert. Es wird über Kieselgur filtriert und der Rückstand mit Ethanol gewaschen. Das Filtrat wird im Vakuum zur Trockne eingeengt. Das Produkt wird ohne weitere Reinigung umgesetzt.

Ausbeute: 263 mg (91% d. Th.)

MS (ESI): m/z = 375 (M+H)⁺; 397 (M+Na)⁺.

### Beispiel 3A

### 1-[(Benzyloxy)carbonyl]-L-prolyl-N⁵-(tert-butoxycarbonyl)-N-{2-[(tert-butoxycarbonyl)amino]-ethyl}-L-ornithinamid

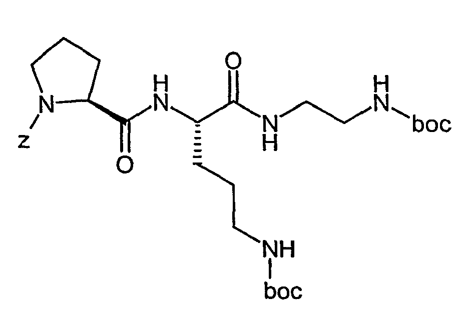

Unter Argon werden 48 mg (0.194 mmol) 1-[(Benzyloxy)carbonyl]-*L*-prolin und 94 mg (0.25 mmol) der Verbindung aus Beispiel 2A in 6 ml Dimethylformamid gelöst. Bei 0°C (Eisbad) werden dann 48 mg (0.25 mmol) EDC und 7.8 mg (0.058 mmol) HOBt zugegeben. Es wird langsam auf RT erwärmt und für 12 h bei RT gerührt. Die Lösung wird im Vakuum eingeengt und der Rückstand mit Dichlormethan aufgenommen und mit gesättigter NatriumhydrogencarbonatLösung, 0.1 N Salzsäure und Wasser gewaschen. Die vereinigten organischen Phasen werden im Vakuum eingeengt und der so erhaltene Feststoff ohne Reinigung weiter umgesetzt.

Ausbeute: 117 mg (95% d. Th.)

LC-MS (Methode 3): Rₜ = 2.36 min.

MS (ESI): m/z = 606 (M+H)⁺

### Beispiel 4A

### L-Prolyl-N⁵-(tert-butoxycarbonyl)-N-{2-[(tert-butoxycarbonyl)amino]ethyl}-L-ornithinamid

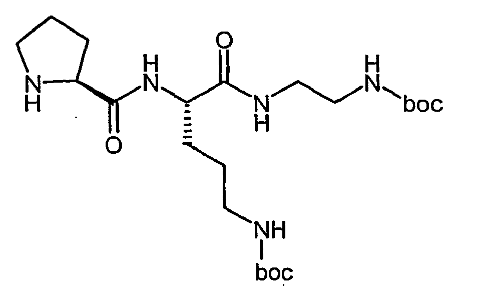

117 mg (0.193 mmol) der Verbindung aus Beispiel 3A werden in 50 ml Ethanol gelöst und mit 20 mg Palladium auf Aktivkohle (10%ig) versetzt. Man hydriert 12 h bei Normaldruck, filtriert über Kieselgur und engt das Filtrat im Vakuum ein. Der so erhaltene Feststoff wird ohne Reinigung weiter umgesetzt.

Ausbeute: 86 mg (94% d. Th.)

MS (ESI): m/z = 472 (M+H)⁺

### Beispiel 5A

### tert-Butyl-{(5S)-5-[(tert-butoxycarbonyl)amino]-6-hydroxyhexyl}carbamat

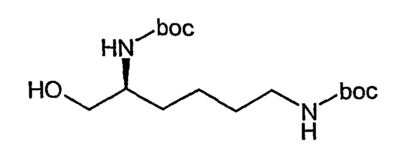

Eine Lösung von 475 mg (0.90 mmol) *N²*,*N⁶*-Bis(*tert*-butoxycarbonyl)-*L*-lysin - *N*-Cyclohexylcyclohexanamin (1:1) in 10 ml Tetrahydrofuran wird bei -10°C mit 91 mg (0.90 mmol) 4-Methylmorpholin und 98 mg (0.90 mmol) Chlorameisensäureethylester versetzt und 30 min gerührt. Bei dieser Temperatur werden 1.81 ml (1.81 mmol) einer 1M Lösung von Lithiumaluminiumhydrid in Tetrahydrofuran langsam zugetropft. Es wird langsam auf RT erwärmt und für 12 h bei RT gerührt. Unter Eiskühlung gibt man vorsichtig 0.1 ml Wasser und 0.15 ml 4.5%ige Natriumhydroxid-Lösung hinzu und rührt weitere 3 h bei RT. Der Ansatz wird filtriert und das Filtrat wird im Vakuum eingeengt. Der Rückstand wird in Essigsäureethylester gelöst, mit Wasser gewaschen, über Magnesiumsulfat getrocknet und erneut im Vakuum zur Trockne eingeengt. Das Produkt wird ohne weitere Reinigung umgesetzt.

Ausbeute: 250 mg (83% d. Th.)

MS (ESI): m/z = 333 (M+H)⁺.

### Beispiel 6A

### (2S)-2,6-Bis[(tert-butoxycarbonyl)amino]hexyl-methansulfonat

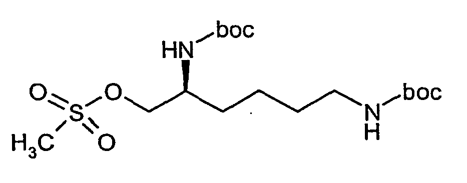

Eine Lösung von 250 mg (0.75 mmol) der Verbindung aus Beispiel 5A in 20 ml Dichlormethan wird mit 103 mg (0.90 mmol) Methansulfonsäurechlorid und 0.21 ml (1.5 mmol) Triethylamin versetzt und für 16 h bei RT gerührt. Es wird mit Dichlormethan verdünnt und zweimal mit 0.1N Salzsäure gewaschen. Die organische Phase wird über Magnesiumsulfat getrocknet und im Vakuum bis zur Trockne eingeengt. Das Produkt wird ohne weitere Reinigung umgesetzt.

Ausbeute: 264 mg (86% d. Th.)

MS (DCI): m/z = 428 (M+NH₄)⁺.

### Beispiel 7A

### tert-Butyl-{(5S)-6-azido-5-[(tert-butoxycarbonyl)amino]hexyl}carbamat

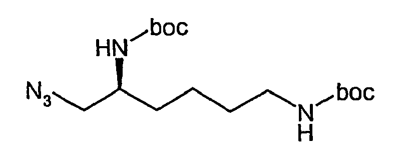

Eine Lösung von 264 mg (0.64 mmol) der Verbindung aus Beispiel 6A in 15 ml Dimethylformamid wird mit 42 mg (0.64 mmol) Natriumazid versetzt und 12 h bei 70°C gerührt. Ein Großteil des Lösungsmittel wird im Vakuum abdestilliert und der Rückstand wird mit Essigsäureethylester verdünnt. Es wird mehrmals mit gesättigter NatriumhydrogencarbonatLösung gewaschen, über Magnesiumsulfat getrocknet und im Vakuum zur Trockne eingeengt. Das Produkt wird ohne weitere Reinigung umgesetzt.

Ausbeute: quant.

MS (ESI): m/z = 358 (M+H)⁺.

### Beispiel 8A

### tert-Butyl-{(5S)-6-amino-5-[(tert-butoxycarbonyl)amino]hexyl}carbamat

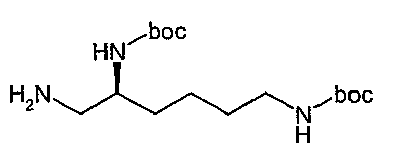

Eine Lösung von 229 mg (0.64 mmol) der Verbindung aus Beispiel 7A in 10 ml Ethanol wird nach Zugabe von 20 mg Palladium auf Aktivkohle (10%ig) 12 h bei RT und Normaldruck hydriert. Es wird über Kieselgur filtriert und der Rückstand mit Ethanol gewaschen. Das Filtrat wird im Vakuum zur Trockne eingeengt. Das Produkt wird ohne weitere Reinigung umgesetzt.

Ausbeute: 161 mg (76% d. Th.)

MS (ESI): m/z = 332 (M+H)⁺.

### Beispiel 9A

### Benzyl-(2S,4R)-2-[({(2S)-2,6-bis[(tert-butoxycarbonyl)amino]hexyl}amino)carbonyl]-4-hydroxypyrrolidin-1-carboxylat

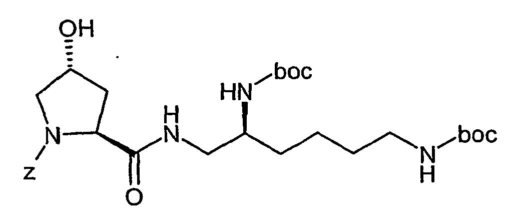

Die Herstellung erfolgt analog zu Beispiel 3A aus 57 mg (0.216 mmol) (4*R*)-1-[(Benzyloxy)carbonyl]-4-hydroxy-*L*-prolin und 93 mg (0.28 mmol) der Verbindung aus Beispiel 8A in 6 ml Dimethylformamid unter Zusatz von 54 mg (0.28 mmol) EDC und 8.7 mg (0.065 mmol) HOBt. Das Produkt wird mittels präparativer RP-HPLC gereinigt (Laufmittel Wasser / Acetonitril Gradient: 90:10 → 5:95).

Ausbeute: 42 mg (34% d. Th.)

LC-MS (Methode 1): Rₜ = 2.08 min.

MS (ESI): m/z = 579 (M+H)⁺

### Beispiel 10A

### (4R)-N-{(2S)-2,6-Bis[(tert-butoxycarbonyl)amino]hexyl}-4-hydroxy-L-prolinamid

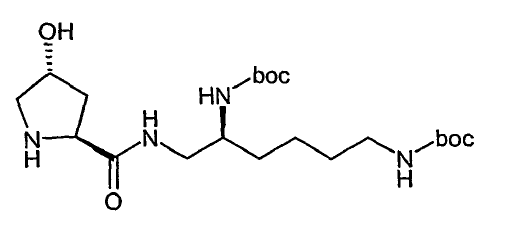

Die Herstellung erfolgt analog Beispiel 4A aus 41 mg (0.071 mmol) der Verbindung aus Beispiel 9A in 20 ml Ethanol unter Zusatz von 7.5 mg Palladium auf Aktivkohle (10%ig). Das Produkt wird ohne weitere Reinigung umgesetzt.

Ausbeute: quant.

MS (ESI): m/z = 445 (M+H)⁺

### Beispiel 11A

### 2-{[(Benzyloxy)carbonyl]amino}ethyl-methansulfonat

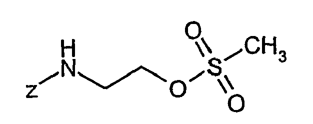

Zu einer Lösung von 16 g (82.0 mmol) Benzyl-(2-hydroxyethyl)carbamat und 16.60 g (64.02 mmol) Triethylamin in 1 1 Dichlormethan werden 11.3 g (98.4 mmol) Methansulfonsäurechlorid hinzugegeben. Das Reaktionsgemisch wird über Nacht bei RT gerührt. Man gibt Wasser hinzu, und die organische Phase wird nacheinander mit Wasser und Natriumchlorid-Lösung gewaschen, über Magnesiumsulfat getrocknet und im Vakuum eingedampft. Der verbleibende Feststoff wird im Hochvakuum getrocknet. Das Rohprodukt wird durch präparative HPLC aufgereinigt.

Ausbeute 7 g (31% d. Th.)

LC-MS (Methode 3): Rₜ = 1.84 min.

MS (ESI): m/z = 273 (M+H)⁺.

### Beispiel 12A

### Benzyl-{2-[(2-hydroxyethyl)amino]ethyl}carbamat

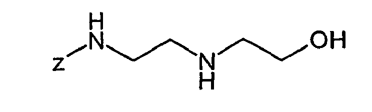

Zu einer Lösung von 226 mg (3.66 mmol) 2-Aminoethanol in 25 ml Acetonitril werden 500 mg (1.83 mmol) 2-{[(Benzyloxy)carbonyl]amino}ethyl-methansulfonat (Beispiel 11A) und 758 mg (5.48 mmol) Kaliumcarbonat hinzugegeben. Das Reaktionsgemisch wird über Nacht bei 50°C gerührt. Das Lösungsmittel wird dann eingedampft und der Rückstand in Dichlormethan aufgenommen. Die organische Phase wird mit Wasser gewaschen, über Magnesiumsulfat getrocknet und eingeengt. Das Rohprodukt wird durch präparative HPLC aufgereinigt.

Ausbeute 131 mg (29% d. Th.)

LC-MS (Methode 3): Rₜ = 0.78 min.

MS (ESI): m/z = 239 (M+H)⁺.

### Beispiel 13A

### Benzyl-[2-({2-[(tert-butoxycarbonyl)(methyl)amino]ethyl}amino)ethyl]carbamat

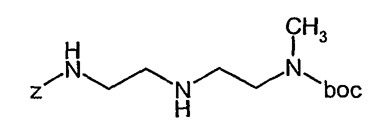

Die Herstellung erfolgt analog zum Beispiel 12A aus 300 mg (1.098 mmol) 2-{[(Benzyloxy)carbonyl]amino}ethyl-methansulfonat (Beispiel 11A), 386 mg (2.19 mmol) *tert-*Butyl-(2-aminoethyl)methylcarbamat und 455 mg (3.30 mmol) Kaliumcarbonat in 10 ml Acetonitril.

Ausbeute: 360 mg (82% d. Th.)

LC-MS (Methode 4): Rₜ = 1.51 min.

MS (ESI): m/z = 352 (M+H)⁺.

### Beispiel 14A

### Benzyl [2-({3-[(tert-butoxycarbonyl)amino]-2-hydroxypropyl}amino)ethyl]carbamat

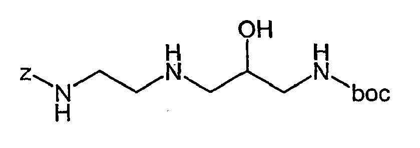

Die Herstellung erfolgt analog zum Beispiel 12A aus 270 mg (0.98 mmol) 2-{[(Benzyloxy)carbonyl]amino}ethyl-methansulfonat (Beispiel 11A), 379 mg (1.98 mmol) *tert*-Butyl-(3-amino-2-hydroxypropyl)carbamat und 409 mg (2.96 mmol) Kaliumcarbonat in 10 ml Acetonitril.

Ausbeute: 209 mg (45% d. Th.)

LC-MS (Methode 2): Rₜ = 1.44 min.

MS (ESI): m/z = 368 (M+H)⁺.

### Beispiel 15A

### 1-tert-Butyl 5-(methoxycarbonyl) N-(tert-butoxycarbonyl)-L-glutamat

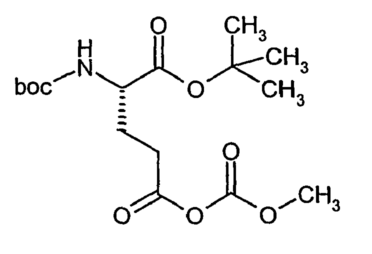

Unter Argon werden 5 g (16.15 mmol) (4*S*)-5-*tert*-Butoxy-4-[(*tert*-butoxycarbonyl)amino]-5-pentansäure und 2.45 ml (17.60 mmol) Triethylamin in 80 ml THF gelöst und auf 0°G abgekühlt. Dazu gibt man 1.68 g (17.77 mmol) Methylchloroformiat und lässt 3 Stunden bei 0°C nachrühren. Die Reaktionsmischung wird über Kieselgur filtriert. Das Filtrat wird direkt umgesetzt.

### Beispiel 16A

### tert-Butyl-N-(tert-butoxycarbonyl)-5-hydroxy-L-norvalinat

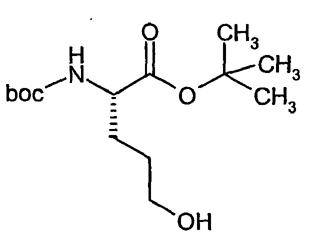

Das Filtrat von (1-*tert*-Butyl-5-(methoxycarbonyl)-*N*-(*tert*-butoxycarbonyl)-*L*-glutamat (Beispiel 15A) wird zu einer Suspension von 1.52 g (40.38 mmol) Natriumborhydrid in 4.5 ml Wasser bei 0°C tropfenweise hinzugegeben. Die Mischung erwärmt sich langsam auf RT und wird über Nacht gerührt. Die Reaktionslösung wird im Vakuum eingeengt und der Rückstand wird zur Aufarbeitung mit Essigsäureethylester und Wasser versetzt. Die organische Phase wird mit gesättigter Natriumchlorid-Lösung gewaschen, über Magnesiumsulfat getrocknet und im Vakuum eingedampft. Das Rohprodukt wird ohne weitere Reinigung umgesetzt.

Ausbeute: 4.5 g (48% d.Th.)

LC-MS (Methode 2): Rₜ = 2.04 min.

MS (ESI): m/z= 290 (M+H)⁺

### Beispiel 17A

### tert-Butyl-N-(tert-butoxycarbonyl)-5-[(methylsulfonyl)oxy]-L-norvalinat

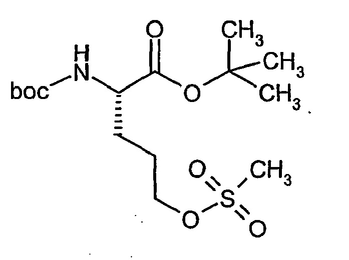

Zu einer Mischung von 4.5 g (7.79 mmol) *tert*-Butyl-*N*-(*tert*-butoxycarbonyl)-5-hydroxy-*L-*norvalinat (Beispiel 16A) und 2.17 ml (5.58 mmol) Triethylamin in 200 ml Dichlormethan werden 1.07 g (9.35 mmol) Methansulfonsäurechlorid hinzugegeben. Die Mischung wird bei RT über Nacht gerührt und dann mit Wasser versetzt. Die organische Phase wird nacheinander mit Wasser und gesättigter Natriumchlorid-Lösung gewaschen, über Magnesiumsulfat getrocknet und im Vakuum eingedampft. Das Rohprodukt wird durch präparative HPLC aufgereinigt.

Ausbeute: quant.

LC-MS (Methode 1): Rₜ = 2.16 min.

MS (ESI): m/z = 368 (M+H)⁺.

### Beispiel 18A

### tert-Butyl-N²-(tert-butoxycarbonyl)-N⁵-{2-[(tert-butoxycarbonyl)amino]ethyl}-L-ornithinat

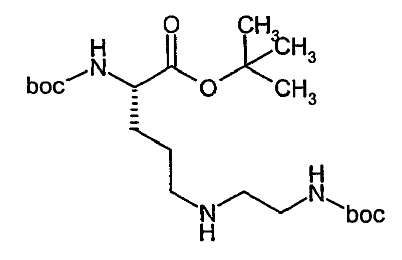

Die Herstellung erfolgt analog zum Beispiel 12A aus 2 g (5.443 mmol) *tert*-Butyl-*N*-(*tert-*butoxycarbonyl)-5-[(methylsulfonyl)oxy]-*L*-norvalinat (Beispiel 17A), 1.78 g (10.89 mmol) *tert-*Butyl-(2-aminoethyl)carbamat und 2.26 g (16.33mmol) Kaliumcarbonat in 100 ml Acetonitril. Das Rohprodukt wird ohne weitere Reinigung umgesetzt.

Ausbeute: 4.2 g (53% d. Th.)

LC-MS (Methode 3): Rₜ = 1.61 min.

MS (ESI): m/z = 432 (M+H)⁺.

### Beispiel 19A

### tert-Butyl 2-{[(2-{[(benzyloxy)carbonyl]amino}ethyl)amino]methyl}piperidin-1-carboxylat

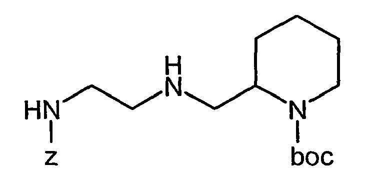

Die Herstellung erfolgt analog zum Beispiel 12A aus 1 g (3.66 mmol) 2-{[(Benzyloxy)carbonyl]-amino}ethyl-methansulfonat (Beispiel 11A), 1.56 g (7.31 mmol) *tert*-Butyl-2-(aminomethyl)-piperidin-1-carboxylat und 1.52 g (10.98 mmol) Kaliumcarbonat in 70 ml Acetonitril.

Ausbeute: 680 mg (45% d. Th.)

LC-MS (Methode 1): Rₜ = 1.47 min.

MS (ESI): m/z = 392 (M+H)⁺.

### Beispiel 20A

### Benzyl-{2-[{(2S)-5-{[(benzyloxy)carbonyl]amino}-2-[(tert-butoxycarbonyl)amino]pentanoyl}(2-hydroxyethyl)amino]ethyl}carbamat

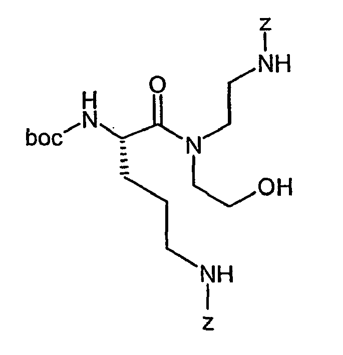

Zu einer Lösung von 169 mg (0.462 mmol) *N⁵*-(Benzyloxy)carbonyl]-*N²*-(*tert*-butoxycarbonyl)-*L-*omithin in 10 ml wasserfreiem DMF werden 193 mg (0.51 mmol) HATU und 0.247 ml (1.39 mmol) *N,N*-Diisopropylethylamin hinzugegeben. Nach 30 min Rühren bei RT werden 116 mg (0.46 mmol) Benzyl-{2-[(2-hydroxyethyl)amino]ethyl}carbamat (Beispiel 12A) hinzugegeben. Das Reaktionsgemisch wird 15 h bei RT gerührt. Das Lösungsmittel wird dann eingedampft und der Rückstand in Essigsäureethylester und Wasser versetzt Die organische Phase wird nacheinander mit 1N Salzsäure und gesättigter wässriger Natriumchlorid-Lösung gewaschen, über Natriumsulfat getrocknet und eingeengt. Das Rohprodukt wird durch präparative HPLC aufgereinigt.

Ausbeute 188 mg (70% d. Th.)

LC-MS (Methode 1): Rₜ = 2.17 min.

MS (ESI): m/z = 587 (M+H)⁺.

### Beispiel 21A

### Benzyl-{(4S)-4-amino-5-[(2-{[(benzyloxy)carbonyl]amino}ethyl)(2-hydroxyethyl)amino]-5-oxopentyl}carbamat Hydrochlorid

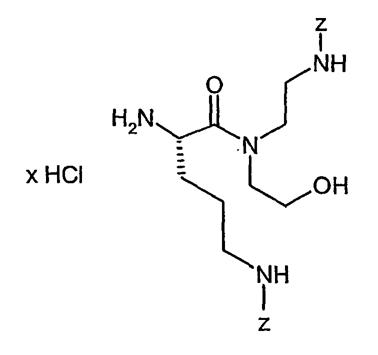

Zu einer Lösung von 176 mg (0.30 mmol) Benzyl-{2-[{(2*S*)-5-{[(benzyloxy)carbonyl]amino}-2-[(*tert*-butoxycarbonyl)amino]pentanoyl}(2-hydroxyethyl)amino]ethyl}carbamat (Beispiel 20A) in 1 ml Dioxan werden 2 ml einer 4 M Chlorwasserstoff-Dioxan-Lösung hinzugegeben. Nach 3 h bei RT wird die Reaktionslösung im Vakuum eingeengt, mehrmals mit Dichlormethan coevaporiert und im Hochvakuum getrocknet. Das Rohprodukt wird ohne weitere Reinigung umgesetzt.

Ausbeute: 160 mg (85% d. Th.)

LC-MS (Methode 1): Rₜ = 1.53 min.

MS (ESI): m/z = 487 (M-HCl+H)⁺.

### Beispiel 22A

### tert-Butyl-3-[(3S)-3-(3-{[(benzyloxy)carbonyl]amino}propyl)-5-(2-hydroxyethyl)-4,9-dioxo-11-phenyl-10-oxa-2,5,8-triazaundecan-1-oyl]piperidin-1-carboxylat

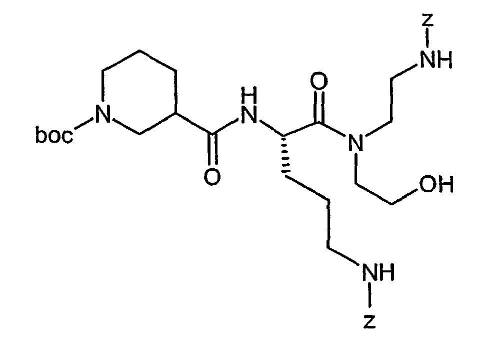

Unter Argon werden 47 mg (0.206 mmol) 1-(*tert*-Butoxycarbonyl)piperidin-3-carbonsäure, 130 mg (0.206 mmol) Benzyl-{(4*S*)-4-amino-5-[(2-{[(benzyloxy)carbonyl]amino}ethyl)(2-hydroxyethyl)-amino]-5-oxopentyl}carbamat Hydrochlorid (Beispiel 21A) und 0.08 ml Triethylamin (0.56 mmol) in 10 ml Dimethylformamid gelöst. Bei 0°C (Eisbad) werden dann 67 mg (0.350 mmol) EDC und 9 mg (0.068 mmol) HOBt zugegeben. Es wird langsam auf RT erwärmt und für 12 h bei RT gerührt. Die Lösung wird im Vakuum eingeengt und der Rückstand wird mit Dichlormethan aufgenommen. Die organische Phase wird nacheinander mit Wasser, 1 N Salzsäure und gesättigter wässriger Natriumchlorid-Lösung gewaschen, über Natriumsulfat getrocknet und eingeengt. Das Rohprodukt wird ohne weitere Reinigung umgesetzt.

Ausbeute: quant.

LC-MS (Methode 1): Rₜ = 2.26 min.

MS (ESI): m/z = 698 (M+H)⁺.

### Beispiel 23A

### Benzyl-{(4S)-5-[(2-{[(benzyloxy)carbonyl]amino}ethyl)(2-hydroxyethyl)amino]-5-oxo-4-[(piperidin-3-ylcarbonyl)amino]pentyl}carbamat Hydrochlorid

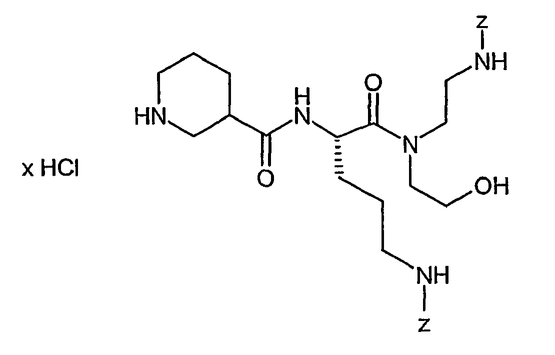

Zu einer Lösung von 180 mg (0.196 mmol) *tert*-Butyl-3-[(3*S*)-3-(3-{[(benzyloxy)carbonyl]-amino}propyl)-5-(2-hydroxyethyl)-4,9-dioxo-11-phenyl-10-oxa-2,5,8-triazaundecan-1-oyl]-piperidin-1-carboxylat (Beispiel 22A) in 1 ml Dioxan werden 2 ml einer 4 M Chlorwasserstoff-Dioxan-Lösung hinzugegeben. Nach 3 h bei RT wird die Reaktionslösung im Vakuum eingeengt, mehrmals mit Dichlormethan coevaporiert und im Hochvakuum getrocknet Das Rohprodukt wird durch präparative HPLC aufgereinigt.

Ausbeute: 18 mg (14% d. Th.)

LC-MS (Methode 2): Rₜ = 1.84 min.

MS (ESI): m/z = 598 (M-HCl+H)⁺.

Analog zu der oben aufgeführten Vorschrift von Beispiel 1A werden die in der folgenden Tabelle aufgeführten Beispiele 24A bis 29A aus den entsprechenden Edukten hergestellt:

| **Bsp.- Nr.** | **Struktur** | **Hergestellt aus** | **Analytische Daten** |
|---|---|---|---|
| **24A** | 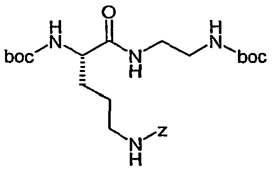 | *N*⁵-[(Benzyloxy)-carbonyl]-*N*²-(*tert*-butoxycarbonyl)-*L*-ornithin und *tert*-Butyl-(2-aminoethyl)-carbamat | LC-MS (Methode 3): Rₜ = 2.33 min. MS (ESI): m/z = 509 (M+H)⁺ |
| **25A** | 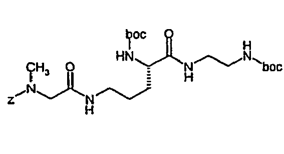 | *N*-[(Benzyloxy)- carbonyl]-*N*-methylglycin und 30A | LC-MS (Methode 2): Rₜ = 2.26 min. MS (ESI): m/z = 579 (M+H)⁺ |
| **26A** | 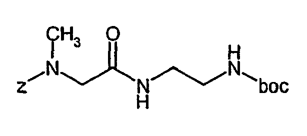 | *N*-[(Benzyloxy)-carbonyl]-*N*-methylglycin und *tert*-Butyl-(2-aminoethyl)-carbamat | LC-MS (Methode 3): Rₜ = 2.03 min. MS (ESI): m/z = 366 (M+H)⁺ |
| **27A** | 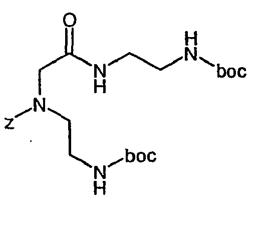 | *N*-[(Benzyloxy)- carbonyl]-*N*-{2-[(*tert*- butoxycarbonyl)- amino]ethyl}glycin amino]ethyl}glycin (CAS 34046-07-6) und *tert*-Butyl-(2-aminoethyl)-carbamat | LC-MS (Methode 3): Rₜ = 2.32 min. MS (ESI): m/z = 495 (M+H)⁺ |
| **28A** | 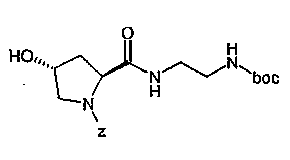 | (4*R*)-1-[(Benzyloxy)- carbonyl]-4-hydroxy-*L*-prolin und *tert*-Butyl-(2-aminoethyl)-carbamat | LC-MS (Methode 2): Rₜ = 1.84 min. MS (ESI): m/z = 408 (M+H)⁺ |
| **29A** | 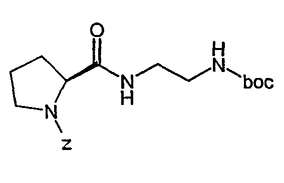 | 1-[(Benzyloxy)- carbonyl]-*L*-prolin und *tert*-Butyl-(2-aminoethyl)-carbamat | LC-MS (Methode 3): Rₜ = 2.1 min. MS (ESI): m/z = 392 (M+H)⁺ |

Analog zu der oben aufgeführten Vorschrift von Beispiel 2A werden die in der folgenden Tabelle aufgeführten Beispiele 30A bis 35A aus den entsprechenden Edukten hergestellt:

| **Bsp.- Nr.** | **Struktur** | **Hergestellt aus** | **Analytische Daten** |
|---|---|---|---|
| **30A** | 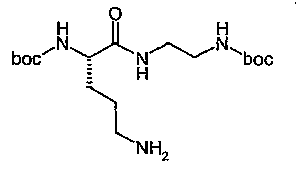 | Beispiel 24A | MS (ESI): m/z = 375 (M+H)⁺ |
| **31A** | 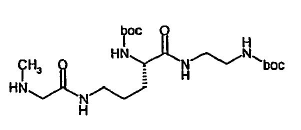 | Beispiel 25A | MS (ESI): m/z = 445 (M+H)⁺ |
| **32A** | 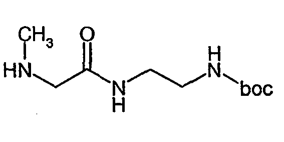 | Beispiel 26A | MS (ESI): m/z = 232 (M+H)⁺ |
| **33A** | 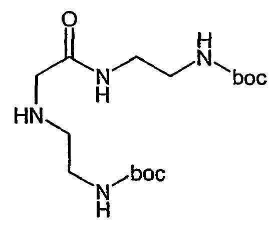 | Beispiel 27A | MS (ESI): m/z = 361 (M+H)⁺ |
| **34A** | 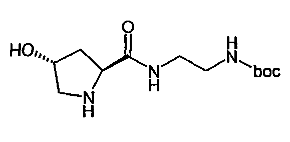 | Beispiel 28A | MS (ESI): m/z = 274 (M+H)⁺ |
| **35A** | 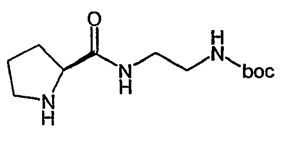 | Beispiel 29A | MS (ESI): m/z = 258 (M+H)⁺ |

### Beispiel 36A

### Benzyl-{2-[{[(8S,11S,14S)-14-[(tert-butoxycarbonyl)amino]-11-{3-[(tert-butoxycarbonyl)amino]-propyl}-5,17-dihydroxy-10,13-dioxo-9,12-diazatricyclo[14.3.1.1^{2,6}]henicosa-1(20),2(21),3,5,16,18-hexaen-8-yl]carbonyl}(2-hydroxyethyl) amino]ethyl}carbamat

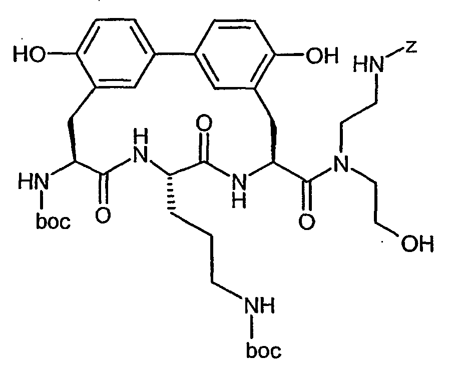

Zu einer Lösung von 30 mg (0.046 mmol) (8*S*,11*S*,14*S*)-14-[(*tert*-Butoxycarbonyl)amino]-11-{3-[(*tert*-butoxycarbonyl)amino]propyl}-5,17-dihydroxy-10,13-dioxo-9,12-diazatricyclo[14.3.1.1^{2,6}]-henicosa-1(20),2(21),3,5,16,18-hexaen-8-carbonsäure (Beispiel 83A aus WO03/106480) in 2 ml wasserfreiem DMF werden 19.2 mg (0.050 mmol) HATU und 0.010 ml (0.137 mmol) *N,N*-Diisopropylethylamin hinzugegeben. Nach 30 min Rühren bei RT werden 12.7 mg (0.046 mmol) Benzyl-{2-[(2-hydroxyethyl)amino]ethyl}carbamat (Beispiel 12A) hinzugegeben. Das Reaktionsgemisch wird 15 h bei RT gerührt. Das Lösungsmittel wird dann eingedampft und der Rückstand in Dichlormethan aufgenommen. Die organische Phase wird mit Wasser gewaschen, über Magnesiumsulfat getrocknet und eingeengt. Das Rohprodukt wird durch präparative HPLC aufgereinigt.

Ausbeute: 8 mg (20% d. Th.)

LC-MS (Methode 2): Rₜ = 2.29 min.

MS (ESI): m/z = 877 (M+H)⁺.

Analog zur Vorschrift des Beispiels 36A werden die in der folgenden Tabelle aufgeführten Beispiele 37A bis 45A hergestellt.

| **Beispiel-Nr.** | **Vorstufe Beispiel** | **Struktur** | **Analytische Daten** |
|---|---|---|---|
| **37A** | Beispiel 83A aus WO 03/106480 + 13A | 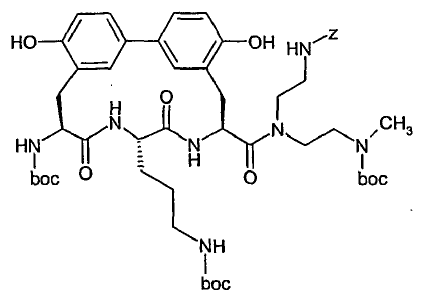 | LC-MS (Methode 1): Rₜ = 2.46 min. MS (ESI): m/z = 990 (M+H)⁺. |
| **38A** | Beispiel 83A aus WO 03/106480 + 14A | 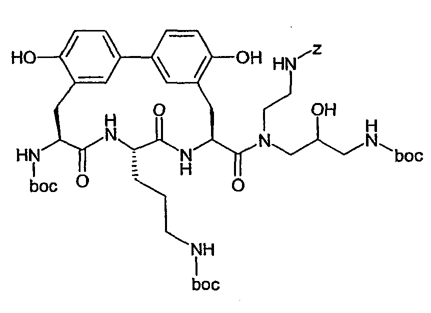 | LC-MS (Methode 3): Rₜ = 2.48 min. MS (ESI): m/z = 1006 (M+H)⁺. |
| **39A** | Beispiel 83A aus WO 03/106480 + 18A | 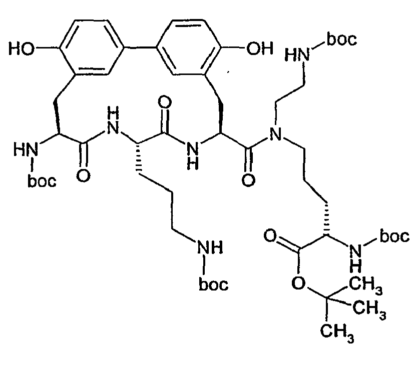 | LC-MS (Methode 1) Rₜ = 1.53 min. MS (ESI): m/z = 1070 (M+H)⁺. |
| **40A** | Beispiel 21A aus WO 03/106480 + 12A | 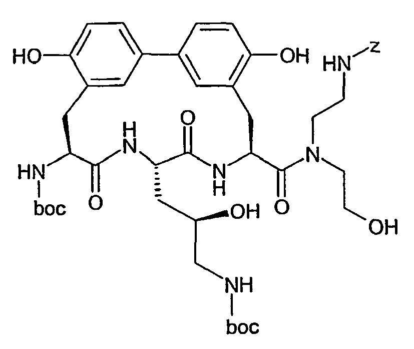 | LC-MS (Methode 1): Rₜ = 2.01 min. MS (ESI): m/z = 893 (M+H)⁺. |
| **41A** | Beispiel 83A aus WO 03/106480 + 19A | 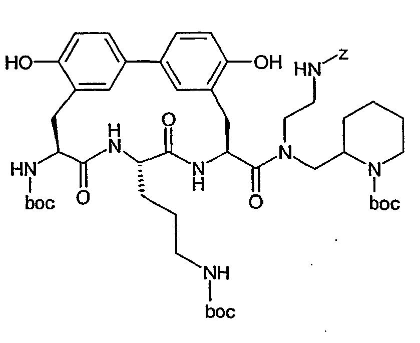 | LC-MS (Methode 3): Rₜ = 2.65 min. MS (ESI): m/z = 1030 (M+H)⁺. |
| **42A** | Beispiel 83A aus WO 03/106480 + 23A | 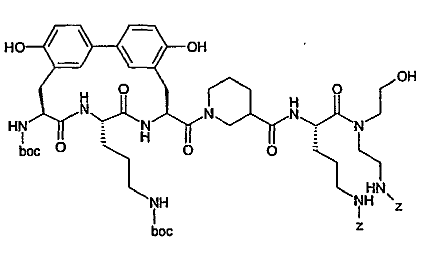 | LC-MS (Methode 2) Rₜ = 2.53 min. MS (ESI): m/z = 1236 (M+H)⁺. |
| **43A** | Beispiel 83A aus WO 03/106480 + 32A | 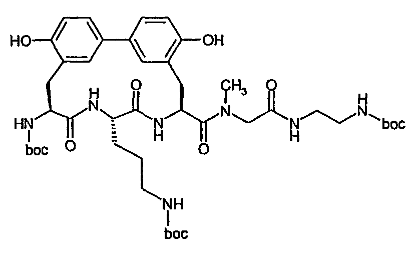 | LC-MS (Methode 3): Rₜ = 2.25 min. MS (ESI): m/z = 870 (M+H)⁺. |
| **44A** | Beispiel 83A aus WO 03/106480 + 33A | 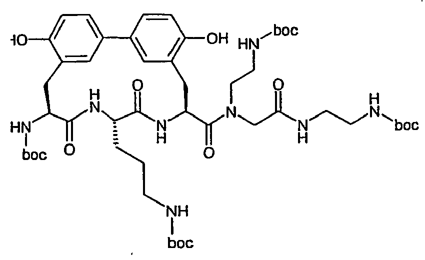 | LC-MS (Methode 2): Rₜ = 2.52 min. MS (ESI): m/z = 999 (M+H)⁺. |
| **45A** | Beispiel 83A aus WO 03/106480 + 31A | 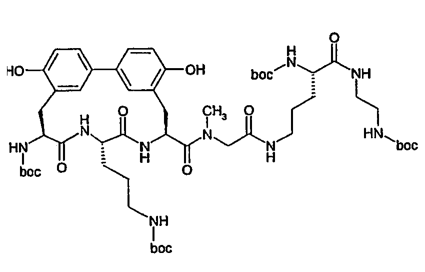 | LC-MS (Methode 1): Rₜ = 2.20 min. MS (ESI): m/z = 1084 (M+H)⁺. |

### Beispiel 46A

### 1-{[(8S,11S,14S)-14-[(tert-Butoxycarbonyl)amino]-11-{3-[(tert-butoxycarbonyl)amino]propyl}-5,17-dihydroxy-10,13-dioxo-9,12-diazatricyclo[14.3.1.1^{2,6}]henicosa-1(20),2(21),3,5,16,18-hexaen-8-yl]carbonyl}-L-prolyl-N⁵-(tert-butoxycarbonyl)-N-{2-[(tert-butoxycarbonyl)amino]ethyl}-L-omithinamid

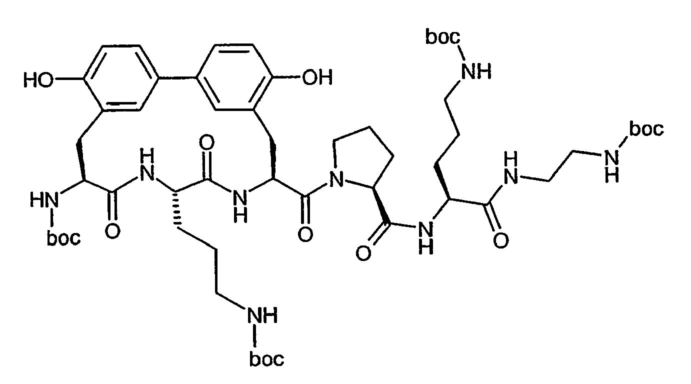

Zu einer Lösung von 30 mg (0.046 mmol) (8*S*,11*S*,14*S*)-14-[(*tert*-Butoxycarbonyl)amino]-11-{3-[(*tert*-butoxycarbonyl)amino]propyl}-5,17-dihydroxy-10,13-dioxo-9,12-diazatricyclo[14.3.1.1^{2,6}]-henicosa-1(20),2(21),3,5,16,18-hexaen-8-carbonsäure (Beispiel 83A aus WO03/106480) in 10 ml wasserfreiem DMF werden bei 0°C 26 mg (0.050 mmol) PyBOP und 0.020 ml (0.14 mmol) *N,N*-Diisopropylethylamin hinzugegeben. Nach 30 min bei 0°C werden 23.7 mg (0.05 mmol) *L-*Prolyl-*N⁵*-(*tert*-butoxycarbonyl)-*N*-{2-[(*tert*-butoxycarbonyl)amino]ethyl}-*L*-ornithinamid (Beispiel 4A) hinzugegeben. Das Reaktionsgemisch wird 15 h bei RT gerührt. Das Lösungsmittel wird dann eingedampft und der Rückstand mit Wasser ausgerührt, abfiltriert und im Vakuum getrocknet. Das Rohprodukt wird chromatographisch an Sephadex-LH20 (Laufmittel: Methanol / Essigsäure (0.25%)) gereinigt.

Ausbeute: 34 mg (66% d. Th.)

LC-MS (Methode 2): Rₜ = 2.49 min.

MS (ESI): m/z = 1110 (M+H)⁺.

Analog zur Vorschrift des Beispiels 46A werden die in der folgenden Tabelle aufgeführten Beispiele 47A bis 50A hergestellt.

| **Beispiel- Nr.** | **Vorstufe Beispiel** | **Struktur** | **Analytische Daten** |
|---|---|---|---|
| **47A** | Beispiel 83A aus WO 03/106480 + 35A | 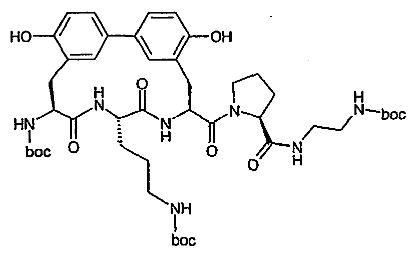 | LC-MS (Methode 3): Rₜ = 2.26 min. MS (ESI): m/z = 896 (M+H)⁺. |
| **48A** | Beispiel 83A aus WO 03/106480 + 10A | 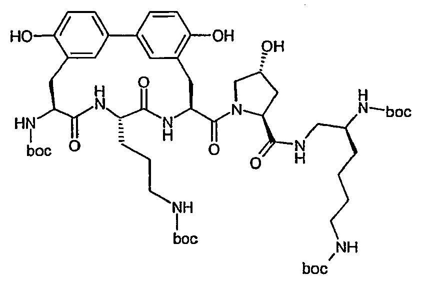 | LC-MS (Methode 1): Rₜ = 2.29 min. MS (ESI): m/z = 1083 (M+H)⁺. |
| **49A** | Beispiel 21A aus WO 03/106480 + 4A | 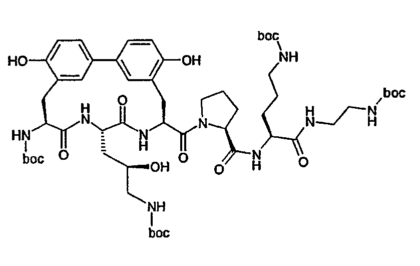 | LC-MS (Methode 3): Rₜ = 2.21 min. MS (ESI): m/z = 1126 (M+H)⁺. |
| **50A** | Beispiel 83A aus WO 03/106480 + 34A | 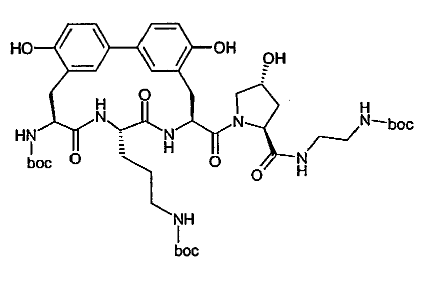 | LC-MS (Methode 3): Rₜ = 2.15 min. MS (ESI): m/z = 912 (M+H)⁺. |

### Beispiel 51A

### tert-Butyl-[2-((2-aminoethyl){[(8S,11S,14S)-14-[(tert-butoxycarbonyl)amino]-11-{3-[(tert-butoxycarbonyl)amino]propyl}-5,17-dihydroxy-10,13-dioxo-9,12-diazatricyclo[14.3.1.1^{2,6}]henicosa-1(20),2(21),3,5,16,18-hexaen-8-yl]carbonyl}amino)ethyl]methylcarbamat Hydroacetat

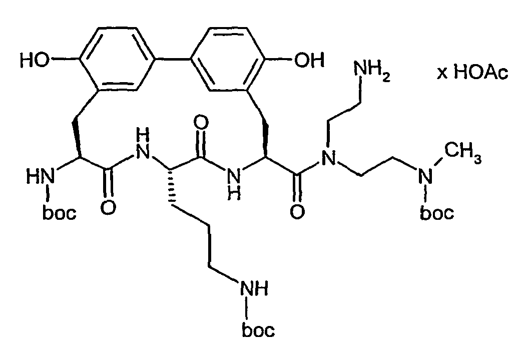

Es werden 11 mg (0.01 mmol) Benzyl [2-({[(8*S*,11*S*,14*S*)-14-[(*tert*-butoxycarbonyl)amino]- 11-{3-[(*tert*-butoxycarbonyl)amino]propyl}-5,17-dihydroxy-10,13-dioxo-9,12-diazatricyclo[14.3.1.1^{2,6}]-henicosa-1(20),2(21),3,5,16,18-hexaen-8-yl]carbonyl} {2-[(*tert*-butoxycarbonyl)(methyl)amino]-ethyl}amino)ethyl]carbamat (Beispiel 37A) in 4 ml Essigsäure/Wasser (4:1) gelöst. Dazu gibt man 5 mg Palladium auf Aktivkohle (10%ig) und hydriert anschließend 15 h bei Normaldruck. Das Reaktionsgemisch wird über vorgewaschenem Kieselgur filtriert und das Filtrat im Vakuum einrotiert. Das Rohprodukt wird ohne weitere Reinigung umgesetzt.

Ausbeute: quant.

LC-MS (Methode 1): Rₜ = 1.80 min.

MS (ESI): m/z = 856 (M-HOAc+H)⁺.

Analog zur Vorschrift des Beispiels 51A werden die in der folgenden Tabelle aufgeführten Beispiele 52A bis 56A hergestellt.

| **Beispiel- Nr.** | **Vorstufe Beispiel** | **Struktur** | **Analytische Daten** |
|---|---|---|---|
| **52A** | 38A | 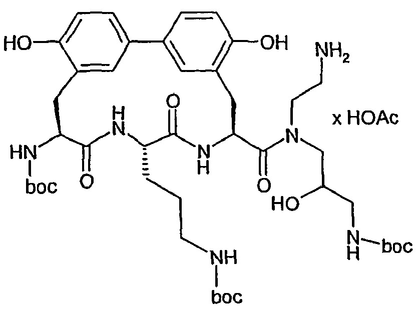 | Das Rohprodukt wird ohne weitere Reinigung umgesetzt. |
| **53A** | 40A | 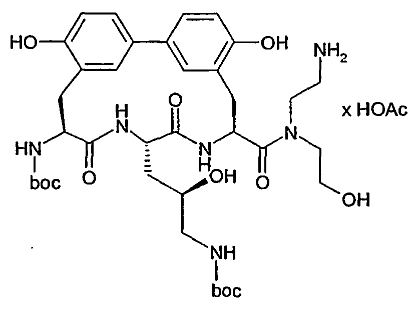 | LC-MS (Methode 1): Rₜ = 1.37 min. MS (ESI): m/z = 759 (M-HOAc+H)⁺. |
| **54A** | 36A | 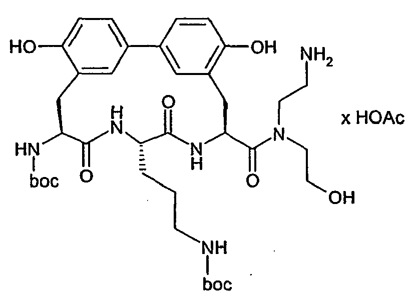 | LC-MS (Methode 2): Rₜ = 1.69 min. MS (ESI): m/z = 742 (M-HOAc+H)⁺. |
| **55A** | 41A | 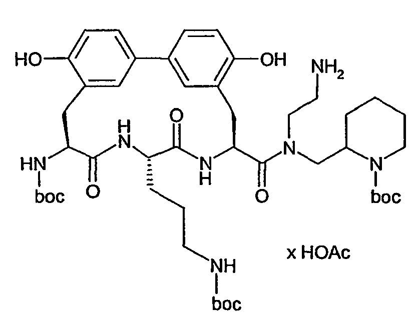 | LC-MS (Methode 1): Rₜ = 1.77 min. MS (ESI): m/z = 896 (M-HOAc+H)⁺. |
| **56A** | 42A | 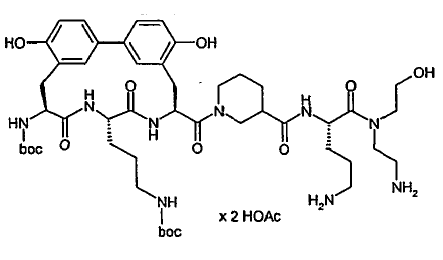 | LC-MS (Methode 1) Rₜ = 1.14 min. MS (ESI): m/z = 968 (M-2HOAc+H)⁺. |

### Ausführungsbeispiele

### Beispiel 1

### 1-{[(8S,11S,14S)-14-Amino-11-(3-aminopropyl)-5,17-dihydroxy-10,13-dioxo-9,12-diazatricyclo[14.3.1.1^{2,6}]henicosa-1(20),2(21),3,5,16,18-hexaen-8-yl]carbonyl}-L-prolyl-N-(2-arninoethyl)-L-omithinamid Tetrahydrochlorid

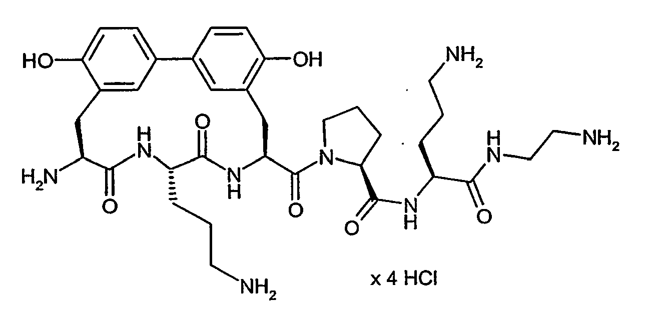

Zu einer Lösung von 26.9 mg (0.024 mmol) der Verbindung aus Beispiel 46A in 1 ml Dioxan werden bei 0°C 0.363 ml einer 4N Chlorwasserstoff-Dioxan-Lösung hinzugegeben. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.

Ausbeute: 20 mg (96% d. Th.)

LC-MS (Methode 5): Rₜ = 1.82 min.

MS (ESI): m/z = 710 (M-4HCl+H)⁺.

¹H-NMR (400 MHz, D₂O): δ = 1.26 (m_{c}, 1H), 1.55-2.1 (m, 10H), 2.27 (m_{c}, 1H), 2.75 (m_{c}, 1H), 2.9-3.2 (m, 7H), 3.3-3.8 (m, 6H), 4.25 (m_{c}, 1H), 4.44 (m_{c}, 2H), 4.7-4.9 (m, 2H, unter D₂O), 6.85-7.0 (m, 3H), 7.27 (s, 1H), 7.34 (d, 1H), 7.4 (d, 1H).

### Beispiel 2

### 1-{[(8S,11S,14S)-14-Amino-11-(3-aminopropyl)-5,17-dihydroxy-10,13-dioxo-9,12-diazatricydo[14.3.1.1^{2.6}]henicosa-1(20),2(21),3,5,16,18-hexaen-8-yl]carbonyl}-L-prolyl-N-(2-aminoethyl)-L-ornithinamid Tetra(hydrotrifluoracetat)

Beispiel 1 als Tetrahydrochlorid-Salz wird durch präparative HPLC (Reprosil ODS-A, Laufmittel Acetonitril / 0.2% wässrige Trifluoressigsäure 5:95 → 95:5) in das Tetra(hydrotrifluoracetat) überführt.

### Beispiel 3

### (8S,11S,14S)-14-Amino-N-(2-aminoethyl)-11-(3-aminopropyl)-5,17-dihydroxy-N-[2-(methylamino)ethyl]-10,13-dioxo-9,12-diazatricyclo[14.3.1.1^{2.6}]henicosa-1(20),2(21),3,5,16,18-hexaen-8-carboxamid Tetrahydrochlorid

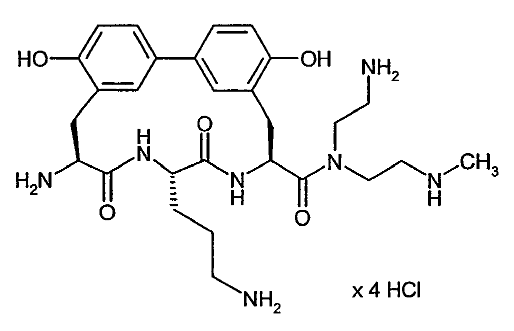

Eine Mischung von 9 mg (0.011 mmol) *tert*-Butyl-[2-((2-aminoethyl){[(8*S*,11*S*,14*S*)-14-[(*tert-*butoxycarbonyl)amino]-11-{3-[(*tert*-butoxycarbonyl)amino]propyl}-5,11-dihydroxy-10,13-dioxo-9,12-diazatricyclo[14.3.1.1^{2,6}]henicosa-1(20),2(21),3,5,16,18-hexaen-8-yl]carbonyl}amino)ethyl]-methylcarbamat (Beispiel 51A) und 1.5 ml einer 4 M Chlorwasserstoff-Dioxan-Lösung wird 20 min bei RT gerührt. Die Reaktionslösung wird eingeengt, mehrmals mit Dichlormethan coevaporiert und im Hochvakuum getrocknet.

Ausbeute: quant.

LC-MS (Methode 1): Rₜ = 0.27 min.

MS (ESI): m/z = 555 (M-4HCl+H)⁺.

¹H-NMR (400 MHz, D₂O): δ = 1.60-1.90 (m, 5H), 2.73 (d, 3H), 2.86-3.11(m, 4H), 3.23-3.38 (m, 4H), 3.50-3.95 (m, 8H), 5.09 (m, 2H), 6.96 (d, 2H), 7.01 (s, 1H), 7.33 (s, 1H), 7.40 (d, 1H), 7.46 (d, 1H).

### Beispiel 4

### 1-{[(8S,11S,14S)-14-Amino-11-(3-aminopropyl)-5,17-dihydroxy-10,13-dioxo-9,12-diazatricyclo[14.3.1.1^{2,6}]henicosa-1(20),2(21),3,5,16,18-hexaen-8-yl]carbonyl}-N-(2-aminoethyl)-L-prolinamid Trishydrochlorid

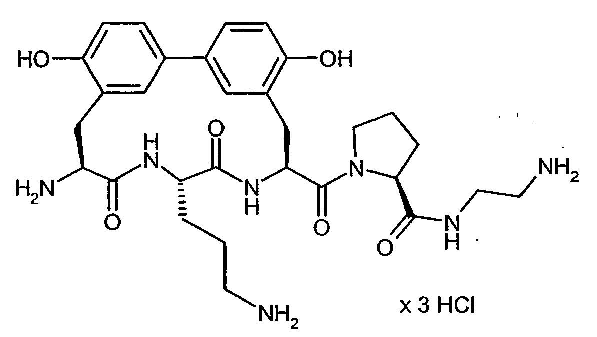

Zu einer Lösung von 26.9 mg (0.024 mmol) der Verbindung aus Beispiel 47A in 1 ml Dioxan werden bei 0°C 0.363 ml einer 4N Chlorwasserstoff-Dioxan-Lösung hinzugegeben. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.

Ausbeute: 20 mg (96% d. Th.)

LC-MS (Methode 5): Rₜ = 1.82 min.

MS (ESI): m/z = 710 (M-3HCl+H)⁺.

¹H-NMR (400 MHz, D₂O): δ = 1.26 (m_{c}, 1H), 1.5-2.1 (m, 8H), 2.26 (m_{c}, 1H), 2.76 (m_{c}, 1H), 2.9-3.2 (m, 7H), 3.3-3.6 (m, 2H), 4.37 (m_{c}, 1H), 4.45 (m_{c}, 1H), 4.7-4.9 (m, 2H, unter D₂O), 6.85-7.0 (m, 3H), 7.27 (s, 1H), 7.34 (d, 1H), 7.4 (d, 1H).

### Beispiel 5

### (8S,11S,14S)-14-Amino-N-{2-[(2-aminoethyl)amino]-2-oxoethyl}-11-(3-aminopropyl)-5,17-dihydroxy-N-methyl-10,13-dioxo-9,12-diazatricyclo[14.3.1.1^{2,6}]henicosa-1 (20),2(21),3,5,16,18-hexaen-8-carboxamid Trishydrochlorid

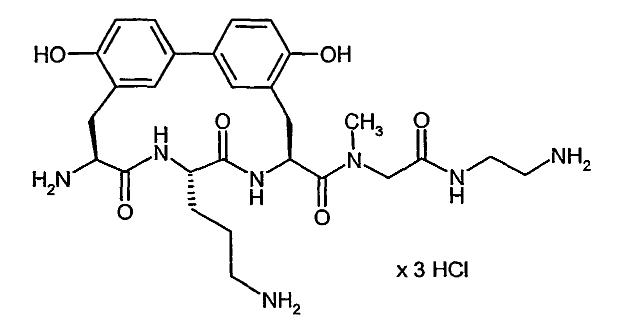

Zu einer Lösung von 19.9 mg (0.023 mmol) der Verbindung aus Beispiel 43A in 1 ml Dioxan werden bei 0°C 0.343 ml einer 4N Chlorwasserstoff-Dioxan-Lösung hinzugegeben. Nach 3 h bei RT wird die Reaktionslösung im Vakuum eingeengt und mehrmals mit Dichlormethan coevaporiert. Der zurückbleibende Feststoff wird im Hochvakuum bis zur Gewichtskonstanz getrocknet.

Ausbeute: 13.6 mg (88% d. Th.)

LC-MS (Methode 5): Rₜ =1.9 min.

MS (ESI): m/z = 570 (M-3HCl+H)⁺.

Analog zur Vorschrift des Beispiels 1 werden die in der folgenden Tabelle aufgeführten Beispiele 5 bis 14 hergestellt, entsprechend der jeweiligen Isolierungsmethode als Hydrochlorid- oder Hydro(trifluoracetat)-Salz.

| **Beispiel- Nr.** | **Vorstufe Beispiel** | **Struktur** | **Analytische Daten** |
|---|---|---|---|
| **6** | 54A | 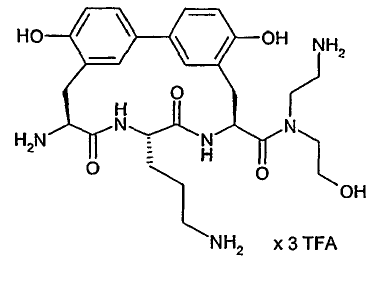 | LC-MS (Methode 5): Rₜ = 1.71 min. MS (ESI): m/z = 543 (M-3TFA+H)⁺. ¹H-NMR (400 MHz, D₂O): δ = 1.40-2.0 (m, 5H), 2.80-3.90 (m, 12H), 5.18 (d, 1H), 6.93-6.96 (m, 2H), 7.01 (s,1H), 7.29 (s, 1H), 7.34-7.45 (m, 2H). |
| **7** | 53A | 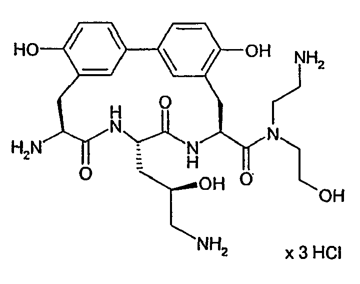 | LC-MS (Methode 5): Rₜ = 1.84 min. MS (ESI): m/z = 559 (M-3HCl+H)⁺. |
| **8** | 52A | 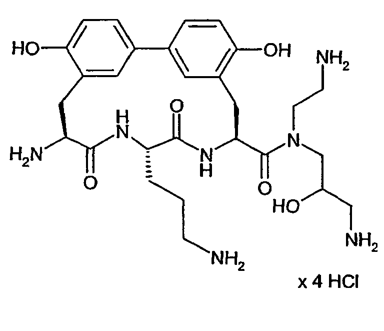 | LC-MS (Methode 1): Rₜ = 0.28 min. MS (ESI): m/z = 572 (M-4HCl+H)⁺. ¹H-NMR (400 MHz, D₂O): δ = 1.20-1.40 (m, 1H), 1.60-2.0 (m, 5H), 2.70-4.30 (m, 21H), 6.98-7.10 (m, 3H), 7.30-7.57 (m, 3H). |
| **9** | 55A | 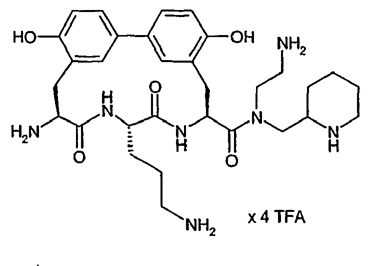 | LC-MS (Methode 6): Rₜ = 1.04 min. MS (ESI): m/z = 596 (M-4TFA+H)⁺. ¹H-NMR (400 MHz, D₂O): δ = 1.30-2.0 (m, 9H), 2.70-4.00 (m, 14H), 4.40 (m, 1H), 5.0 (dd_{c}, 1H), 6.80-6.71 (m, 3H), 7.00-7.20 (m, 3H). |
| **10** | 56A | 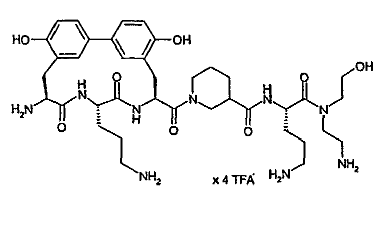 | LC-MS (Methode 6): Rₜ = 2.06 min. MS (ESI): m/z = 768 (M-4TFA+H)⁺. |
| **11** | 39A | 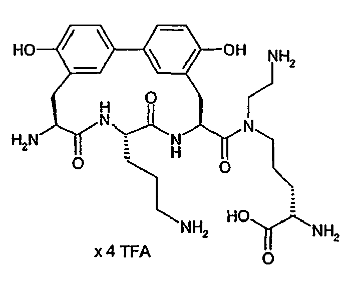 | LC-MS (Methode 5): Rₜ = 1.85 min. MS (ESI): m/z = 614 (M-4TFA+H)⁺. |
| **12** | 48A | 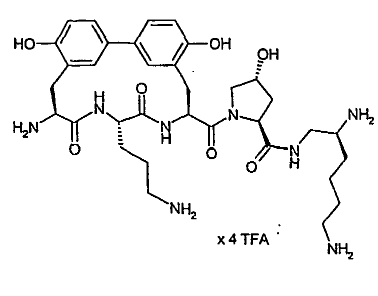 | LC-MS (Methode 5): Rₜ = 1.91 min. MS (ESI): m/z = 683 (M-4TFA+H)⁺. |
| **13** | 49A | 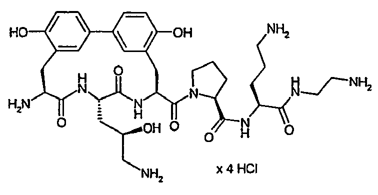 | LC-MS (Methode 5): Rₜ = 1.89 min. MS (ESI): m/z = 726 (M-4HCl+H)⁺. |
| **14** | 44A | 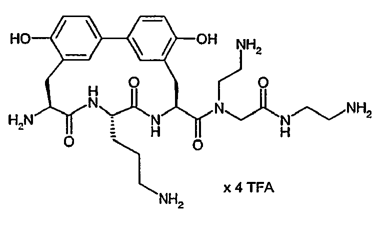 | MS (ESI): m/z = 599 (M-4TFA+H)⁺. ¹H-NMR (400 MHz, D₂O): δ = 1.55-1.9 (m, 4H), 2.76 (m_{c}, 1H), 2.9-3.35 (m, 10H), 3.4-3.6 (m, 2H), 3.86 (m_{c}, 1H), 4.11 (m_{c}, 1H), 4.34 (m_{c}, 1H), 4.43 (m_{c}, 1H), 4.7-4.9 (m, 1H, unter D₂O), 5.14 (m_{c}, 1H), 6.85-7.0 (m, 3H), 7.2-7.45 (m, 3H). |
| **15** | 45A | 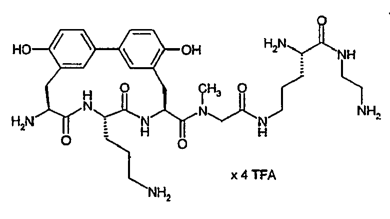 | MS (ESI): m/z = 684 (M- 4TFA+H)⁺. |

### B. Bewertung der physiologischen Wirksamkeit

### Verwendete Abkürzungen:

- AMP: Adenosinmonophosphat
- ATP: Adenosintriphosphat
- BHI Medium: Brain heart infusion medium
- CoA: Coenzym A
- DMSO: Dimethylsulfoxid
- DTT: Dithiothreitol
- EDTA: Ethylendiamintetraessigsäure
- KCl: Kaliumchlorid
- KH₂PO₄: Kaliumdihydrogenphosphat
- MgSO₄: Magnesiumsulfat
- MHK: Minimale Hemmkonzentration
- MTP: Mikrotiterplatte
- NaCl: Natriumchlorid
- Na₂HPO₄: Dinatriumhydrogenphosphat
- NH₄Cl: Ammoniumchlorid
- NTP: Nukleotidtriphosphat
- PBS: Phosphat Buffered Saline
- PCR: Polymerase Chain Reaction
- PEG: Polyethylenglykol
- PEP: Phosphoenolpyruvat
- Tris: Tris[hydroxymethyl]aminomethan

Die *in vitro*-Wirkung der erfindungsgemäßen Verbindungen kann in folgenden Assays gezeigt werden:

### In vitro Transkription-Translation mit E. coli Extrakten

Zur Herstellung eines S30-Extraktes werden logarithmisch wachsende *Escherichia coli* MRE 600 (M. Müller; University Freiburg) geerntet, gewaschen und wie beschrieben für den *in vitro* Transkriptions-Translations-Test eingesetzt (Müller, M. and Blobel, G. Proc Natl Acad Sci U S A (1984) 81, pp.7421-7425).

Dem Reaktionsmix des *in vitro* Transkriptions-Translations-Tests werden zusätzlich 1 µl cAMP (11.25 mg/ml) je 50 µl Reaktionsmix zugegeben. Der Testansatz beträgt 105 µl, wobei 5 µl der zu testenden Substanz in 5%igem DMSO vorgelegt werden. Als Transkriptionsmatrize werden 1 µg/100µl Ansatz des Plasmides pBESTLuc (Promega, Deutschland) verwendet. Nach Inkubation für 60 min bei 30°C werden 50 µl Luziferinlösung (20 mM Tricine, 2.67 mM MgSO₄, 0.1 mM EDTA, 33.3 mM DTT pH 7.8, 270 µM CoA, 470 µM Luziferin, 530 µM ATP) zugegeben und die entstehende Biolumineszenz für 1 Minute in einem Luminometer gemessen. Als IC₅₀ wird die Konzentration eines Inhibitors angegeben, die zu einer 50%igen Inhibition der Translation von Firefly Luziferase führt.

### In vitro Transkription-Translation mit S. aureus Extrakten

### Konstruktion eines S. aureus Luziferase Reporterplasmids

Zur Konstruktion eines Reporterplasmids, welches in einem *in vitro* Transkriptions-Translations-Assay aus S. *aureus* verwendet werden kann, wird das Plasmid pBESTluc (Promega Corporation, USA) verwendet. Der in diesem Plasmid vor der Firefly Luziferase vorhandene *E. coli tac* Promoter wird gegen den *capA1* Promoter mit entsprechender Shine-Dalgarno Sequence aus S. *aureus* ausgetauscht. Dazu werden die Primer CAPFor 5'-CGGCC-AAGCTTACTCGGATCCAGAGTTTGCAAAATATACAGGGGATTATATATAATGGAAAAC AAGAAAGGAAAATAGGAGGTTTATATGGAAGACGCCA-3' und CAPRev 5'-GTCATCGTCGGGAAGACCTG-3' verwendet. Der Primer CAPFor enthält den *capA1* Promotor, die Ribosomenbindestelle und die 5'-Region des Luziferase Gens. Nach PCR unter Verwendung von pBESTluc als Template kann ein PCR-Produkt isoliert werden, welches das Firefly Luziferase Gen mit dem fusionierten *capA1* Promotor enthält. Dieses wird nach einer Restriktion mit ClaI und HindIII in den ebenfalls mit ClaI und HindIII verdauten Vektor pBESTluc ligiert. Das entstandene Plasmid pla kann in *E. coli* repliziert werden und als Template im *S*. *aureus in vitro* Transkriptions-Translations-Test verwendet werden.

### Herstellung von S30 Extrakten aus S. aureus

Sechs Liter BHI Medium werden mit einer 250 ml Übernachtkultur eines S. aureus Stammes inokuliert und bei 37°C bis zu einer OD600nm von 2-4 wachsen gelassen. Die Zellen werden durch Zentrifugation geerntet und in 500 ml kaltem Puffer A (10 mM Tris-acetat, pH 8.0, 14 mM Magnesiumacetat, 1 mM DTT, 1 M KCl) gewaschen. Nach erneutem Abzentrifugieren werden die Zellen in 250 ml kaltem Puffer A mit 50 mM KCl gewaschen und die erhaltenen Pellets bei -20°C für 60 min eingefroren. Die Pellets werden in 30 bis 60 min auf Eis aufgetaut und bis zu einem Gesamtvolumen von 99 ml in Puffer B (10 mM Tris-acetat, pH 8.0, 20 mM Magnesiumacetat, 1 mM DTT, 50 mM KCl) aufgenommen. Je 1.5 ml Lysostaphin (0.8 mg/ml) in Puffer B werden in 3 vorgekühlte Zentrifugenbecher vorgelegt und mit je 33 ml der Zellsuspension vermischt. Die Proben werden für 45 bis 60 min bei 37°C unter gelegentlichem Schütteln inkubiert, bevor 150 µl einer 0.5 M DTT Lösung zugesetzt werden. Die lysierten Zellen werden bei 30.000 x g 30 min bei 4°C abzentrifugiert. Das Zellpellet wird nach Aufnahme in Puffer B unter den gleichen Bedingungen nochmals zentrifugiert und die gesammelten Überstände werden vereinigt. Die Überstände werden nochmals unter gleichen Bedingungen zentrifugiert und zu den oberen 2/3 des Überstandes werden 0.25 Volumen Puffer C (670 mM Tris-acetat, pH 8.0, 20 mM Magnesiumacetat, 7 mM Na₃-Phosphoenolpyruvat, 7 mM DTT, 5.5 mM ATP, 70 µM Aminosäuren (complete von Promega), 75 µg Pyruvatkinase (Sigma, Deutschland))/ml gegeben. Die Proben werden für 30 min bei 37°C inkubiert. Die Überstände werden über Nacht bei 4°C gegen 2 1 Dialysepuffer (10 mM Tris-acetat, pH 8.0, 14 mM Magnesiumacetat, 1 mM DTT, 60 mM Kaliumacetat) mit einem Pufferwechsel in einem Dialyseschlauch mit 3500 Da Ausschluss dialysiert. Das Dialysat wird auf eine Proteinkonzentration von etwa 10 mg/ml konzentriert, indem der Dialyseschlauch mit kaltem PEG 8000 Pulver (Sigma, Deutschland) bei 4°C bedeckt wird. Die S30 Extrakte können aliquotiert bei -70°C gelagert werden.

### Bestimmung der IC₅₀ im S. aureus in vitro Transcriptions-Translations-Assay

Die Inhibition der Proteinbiosynthese der Verbindungen kann in einem *in vitro* Transkriptions-Translations-Assay gezeigt werden. Der Assay beruht auf der zellfreien Transkription und Translation von Firefly Luziferase unter Verwendung des Reporterplasmids pla als Template und aus *S*. *aureus* gewonnenen zellfreien S30 Extrakten. Die Aktivität der entstandenen Luziferase kann durch Lumineszenzmessung nachgewiesen werden.

Die Menge an einzusetzenden S30 Extrakt bzw. Plasmid pla muss für jede Präparation erneut ausgetestet werden, um eine optimale Konzentration im Test zu gewährleisten. 3 µl der zu testenden Substanz gelöst in 5% DMSO werden in eine MTP vorgelegt. Anschließend werden 10 µl einer geeignet konzentrierten Plasmidlösung pla zugegeben. Anschließend werden 46 µl eines Gemisches aus 23 µl Premix (500 mM Kaliumacetat, 87.5 mM Tris-acetat, pH 8.0, 67.5 mM Ammoniumacetat, 5 mM DTT, 50 µg Folsäure/ml, 87.5 mg PEG 8000/ml, 5 mM ATP, 1.25 mM je NTP, 20 µM je Aminosäure, 50 mM PEP (Na₃-Salz), 2.5 mM cAMP, 250 µg je *E*. *coli* tRNA/ml) und 23 µl einer geeigneten Menge S. *aureus* S30 Extrakt zugegeben und vermischt. Nach Inkubation für 60 min bei 30°C werden 50 µl Luziferinlösung (20 mM Tricine, 2.67 mM MgSO₄, 0.1 mM EDTA, 33.3 mM DTT pH 7.8, 270 µM CoA, 470 µM Luziferin, 530 µM ATP) und die entstehende Biolumineszenz für 1 min in einem Luminometer gemessen. Als IC₅₀ wird die Konzentration eines Inhibitors angegeben, die zu einer 50%igen Inhibition der Translation von Firefly Luziferase führt.

### Bestimmung der Minimalen Hemmkonzentration (MHK)

Die minimale Hemmkonzentration (MHK) ist die minimale Konzentration eines Antibiotikums, mit der ein Testkeim in seinem Wachstum über 18-24 h inhibiert wird. Die Hemmstoffkonzentration kann dabei nach mikrobiologischen Standardverfahren bestimmt werden (siehe z.B. The National Committee for Clinical Laboratory Standards. Methods for dilution antimicrobial susceptibility tests for bacteria that grow aerobically; approved standard-fifth edition. NCCLS document M7-A5 [ISBN 1-56238-394-9]. NCCLS, 940 West Valley Road, Suite 1400, Wayne, Pennsylvania 19087-1898 USA, 2000). Die MHK der erfindungsgemäßen Verbindungen wird im Flüssigdilutionstest im 96er-Mikrotiter-Platten-Maßstab bestimmt. Die Bakterienkeime werden in einem Minimalmedium (18.5 mM Na₂HPO₄, 5.7 mM KH₂PO₄, 9.3 mM NH₄Cl, 2.8 mM MgSO₄, 17.1 mM NaCl, 0.033 µg/ml Thiaminhydrochlorid, 1.2 µg/ml Nicotinsäure, 0.003 µg/ml Biotin, 1% Glucose, 25 µg/ml von jeder proteinogenen Aminosäure mit Ausnahme von Phenylalanin; [H.-P. Kroll; unveröffentlicht]) unter Zusatz von 0.4% BH-Bouillon kultiviert (Testmedium). Im Fall von *Enterococcus faecium* L4001 wird dem Testmedium hitzeinaktiviertes fötales Kälberserum (FCS; GibcoBRL, Deutschland) in einer Endkonzentration von 10% zugesetzt. Übernachtkulturen der Testkeime werden auf eine OD₅₇₈ von 0.001 (im Falle der Enterokokken auf 0.01) in frisches Testmedium verdünnt und 1:1 mit Verdünnungen der Testsubstanzen (Verdünnungsstufen 1:2) in Testmedium inkubiert (200 µl Endvolumen). Die Kulturen werden bei 37°C für 18-24 Stunden inkubiert; Enterokokken in Gegenwart von 5% CO₂.

Die jeweils niedrigste Substanzkonzentration, bei der kein sichtbares Bakterienwachstum mehr auftritt, wird als MHK definiert.

### Alternative Bestimmungsmethode der Minimalen Hemmkonzentration (MHK)

Die minimale Hemmkonzentration (MHK) ist die minimale Konzentration eines Antibiotikums, mit der ein Testkeim in seinem Wachstum über 18-24 h inhibiert wird. Die Hemmstoffkonzentration kann dabei nach mikrobiologischen Standardverfahren mit modifiziertem Medium im Rahmen eines Agardilutionstests bestimmt werden (siehe z.B. The National Committee for Clinical Laboratory Standards. Methods for dilution antimicrobial susceptibility tests for bacteria that grow aerobically; approved standard-fifth edition. NCCLS document M7-A5 [ISBN 1-56238-394-9]. NCCLS, 940 West Valley Road, Suite 1400, Wayne, Pennsylvania 19087-1898 USA, 2000). Die Bakterienkeime werden auf 1.5%igen Agarplatten kultiviert, die 20% defibriniertes Pferdeblut enthalten. Die Testkeime, die über Nacht auf Columbia-Blutagarplatten (Becton-Dickinson) inkubiert werden, werden in PBS verdünnt, auf eine Keimzahl von ca. 5x10⁵ Keime/ml eingestellt und auf Testplatten getropft (1-3 µl). Die Testsubstanzen enthalten unterschiedliche Verdünnungen der Testsubstanzen (Verdünnungsstufen 1:2). Die Kulturen werden bei 37°C für 18-24 Stunden in Gegenwart von 5% CO₂ inkubiert.

Die jeweils niedrigste Substanzkonzentration, bei der kein sichtbares Bakterienwachstum mehr auftritt, wird als MHK definiert und in µg/ml angegeben.

**Tabelle A (mit Vergleichsbeispiel Biphenomycin B)**

| **Bsp.-Nr.** | **MHK** | **MHK** | **MHK** | **IC₅₀** |
|---|---|---|---|---|
| | ***S. aureus* 133** | ***S. aureus* T17** | ***E. faecium* L4001** | ***S. aureus* 133 Translation** |
| **1** | 1.0 | 4.0 | 2.0 | 0.7 |
| **3** | 2.0 | 4.0 | 8.0 | 0.8 |
| **6** | 2.0 | 4.0 | 4.0 | 0.7 |
| **8** | 1.0 | 4.0 | 16.0 | 0.25 |
| **9** | 4.0 | 2.0 | 32 | 0.33 |
| **Biphenomycin B** | <0.03 | >32 | 0.5 | 1.5 |

| | | | | |
|---|---|---|---|---|
| Konzentrationsangaben: MHK in µg/ml; IC₅₀ in µM. | | | | |

### Systemische Infektion mit S. aureus 133

Die Eignung der erfindungsgemäßen Verbindungen zur Behandlung von bakteriellen Infektionen kann in verschiedenen Tiermodellen gezeigt werden. Dazu werden die Tiere im allgemeinen mit einem geeigneten virulenten Keim infiziert und anschließend mit der zu testenden Verbindung, die in einer an das jeweilige Therapiemodell angepassten Formulierung vorliegt, behandelt. Speziell kann die Eignung der erfindungsgemäßen Verbindungen zur Behandlung von bakteriellen Infektionen in einem Sepsismodell an Mäusen nach Infektion mit S. *aureus* demonstriert werden.

Dazu werden *S. aureus* 133 Zellen über Nacht in BH-Bouillon (Oxoid, Deutschland) angezüchtet. Die Übernachtkultur wurde 1:100 in frische BH-Bouillon verdünnt und für 3 Stunden hochgedreht. Die in der logarithmischen Wachstumsphase befindlichen Bakterien werden abzentrifugiert und zweimal mit gepufferter, physiologischer Kochsalzlösung gewaschen. Danach wird am Photometer (Dr. Lange LP 2W) eine Zellsuspension in Kochsalzlösung mit einer Extinktion von 50 Einheiten eingestellt. Nach einem Verdünnungsschritt (1:15) wird diese Suspension 1:1 mit einer 10%-igen Mucinsuspension gemischt. Von dieser Infektionslösung wird 0.2 ml/20 g Maus i.p. appliziert. Dies entspricht einer Zellzahl von etwa 1-2 x 10⁶ Keimen/Maus. Die i.v.-Therapie erfolgt 30 Minuten nach der Infektion. Für den Infektionsversuch werden weibliche CFW1-Mäuse verwendet. Das Überleben der Tiere wird über 6 Tage protokolliert. Das Tiermodell ist so eingestellt, daß unbehandelte Tiere innerhalb von 24 h nach der Infektion versterben. Für die Beispielverbindung 2 konnte in diesem Modell eine therapeutische Wirkung von ED₁₀₀ = 1.25 mg/kg demonstriert werden.

### Bestimmung der Spontanresistenzfrequenzen gegen S. aureus

Die Spontanresistenzraten der erfindungsgemäßen Verbindungen werden wie folgt bestimmt: die Bakterienkeime werden in 30 ml eines Minimalmediums (18.5 mM Na₂HPO₄, 5.7 mM KH₂PO₄, 9.3 mM NH₄Cl, 2.8 mM MgSO₄, 17.1 mM NaCl, 0.033 µg/ml Thiaminhydrochlorid, 1.2 µg/ml Nicotinsäure, 0.003 µg/ml Biotin, 1% Glucose, 25 µg/ml von jeder proteinogenen Aminosäure unter Zusatz von 0,4% BH Bouillon) bei 37°C über Nacht kultiviert, 10 min bei 6.000xg abzentrifugiert und in 2 ml phosphat-gepufferter physiologischer NaCl-Lösung resuspendiert (ca. 2x10⁹ Keime/ml). 100 µl dieser Zellsuspension bzw. 1:10 und 1:100 Verdünnungen werden auf vorgetrockneten Agarplatten (1.5% Agar, 20% defibriniertes Pferdeblut bzw. 1.5% Agar, 20% Rinderserum in 1/10 Müller-Hinton-Medium verdünnt mit PBS), welche die zu testende erfindungsgemäße Verbindung in einer Konzentration entsprechend 5xMHK bzw. 10xMHK enthalten, ausplattiert und 48 h bei 37°C bebrütet. Die entstehenden Kolonien (cfu) werden ausgezählt.

### Isolierung der Biphenomycin-resistenten S. aureus Stämme RN4220Bi^{R} und T17

Der *S. aureus* Stamm RN4220Bi^{R} wird *in vitro* isoliert. Dazu werden jeweils 100 µl einer S. *aureus* RN4220 Zellsuspension (ca. 1.2x10⁸ cfu/ml) auf einer antibiotikafreien Agarplatte (18.5 mM Na₂HPO₄, 5.7 mM KH₂PO₄, 9.3 mM NH₄Cl, 2.8 mM MgSO₄, 17.1 mM NaCl, 0.033 µg/ml Thiaminhydrochlorid, 1.2 µg/ml Nicotinsäure, 0.003 µg/ml Biotin, 1% Glucose, 25 µg/ml von jeder proteinogenen Aminosäure unter Zusatz von 0.4% BH-Bouillon und 1 % Agarose) und einer Agarplatte, die 2 µg/ml Biphenomycin B (10xMHK) enthält, ausplattiert und über Nacht bei 37°C bebrütet. Während auf der antibiotikafreien Platte ca. 1x10⁷ Zellen wachsen, wachsen auf der antibiotikahaltigen Platte ca. 100 Kolonien, entsprechend einer Resistenzfrequenz von 1x10⁻⁵. Einige der auf der antibiotikahaltigen Platte gewachsenen Kolonien werden auf MHK gegen Biphenomycin B getestet. Eine Kolonie mit einer MHK > 50 µM wird zur weiteren Verwendung ausgewählt und der Stamm mit RN4220Bi^{R} bezeichnet.

Der S. *aureus* Stamm T17 wird *in vivo* isoliert. CFW1-Mäuse werden mit 4x10⁷ *S*. *aureus* 133 - Zellen pro Maus intraperitoneal infiziert. 0.5 Std. nach der Infektion werden die Tiere mit 50 mg/kg Biphenomycin B intravenös behandelt. Den überlebenden Tieren werden am Tag 3 nach der Infektion die Nieren entnommen. Nach dem Homogenisieren der Organe werden die Homogenate, wie bei RN4220Bi^{R} beschrieben, auf antibiotikafreien und antibiotikahaltigen Agarplatten, ausplattiert und über Nacht bei 37°C bebrütet. Etwa die Hälfte der aus der Niere isolierten Kolonien zeigen ein Wachstum auf den antibiotikahaltigen Platten (2.2x10⁶ Kolonien), was die Anreicherung von Biphenomycin B resistenten S. *aureus* Zellen in der Niere der behandelten Tiere belegt. Ca. 20 dieser Kolonien werden auf MHK gegen Biphenomycin B getestet und eine Kolonie mit einer MHK > 50 µM wird zur Weiterkultivierung ausgewählt und der Stamm mit T17 bezeichnet.

### C. Ausführungsbeispiele für pharmazeutische Zusammensetzungen

Die erfindungsgemäßen Verbindungen können folgendermaßen in pharmazeutische Zubereitungen überführt werden:

### Intravenös applizierbare Lösung:

### Zusammensetzung:

1 mg der Verbindung von Beispiel 1, 15 g Polyethylenglykol 400 und 250 g Wasser für Injektionszwecke.

### Herstellung:

Die erfindungsgemäße Verbindung wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 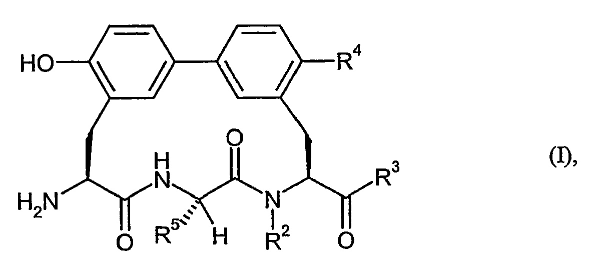 in welcher
R⁵ gleich eine Gruppe der Formel 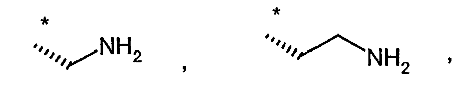 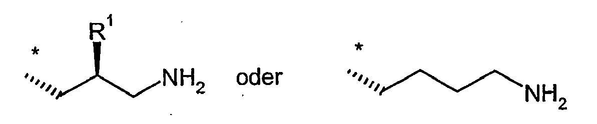 ist,
wobei
* die Anknüpfstelle an das Kohlenstoffatom ist,
R¹ gleich Wasserstoff oder Hydroxy ist,
R² gleich Wasserstoff, Methyl oder Ethyl ist,
R³ gleich eine Gruppe der Formel 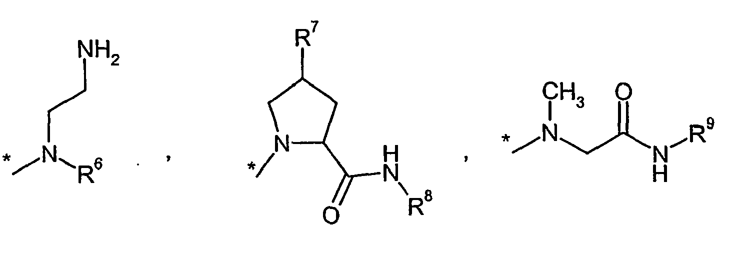 oder 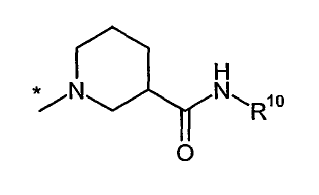 ist,
wobei
* die Anknüpfstelle an das Stickstoffatom ist,
R⁶ gleich eine Gruppe der Formel 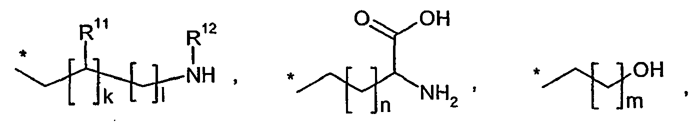 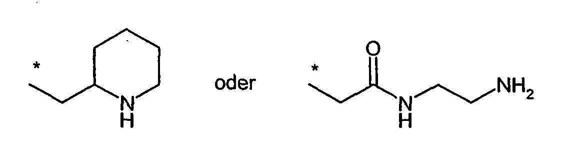 ist,
worin
* die Anknüpfstelle an das Stickstoffatom ist,
R¹¹ gleich Wasserstoff, Amino oder Hydroxy ist,
R¹² gleich Wasserstoff oder Methyl ist,
k eine Zahl 0 oder 1 ist,
l eine Zahl 1, 2, 3 oder 4 ist,
und
m und n unabhängig voneinander eine Zahl 1, 2 oder 3 sind,
R⁷ gleich Wasserstoff, Amino oder Hydroxy ist,
R⁸, R⁹ und R¹⁰ unabhängig voneinander eine Gruppe der Formel 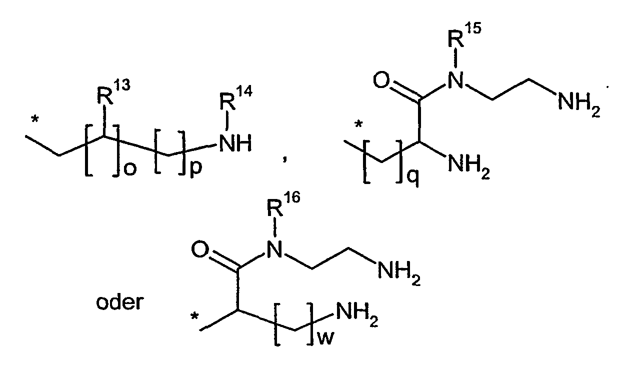 sind,
worin
* die Anknüpfstelle an das Stickstoffatom ist,
R¹³ gleich Wasserstoff, Amino oder Hydroxy ist,
R¹⁴ gleich Wasserstoff oder Methyl ist,
R¹⁵ und R¹⁶ unabhängig voneinander Wasserstoff, Aminoethyl oder Hydroxyethyl sind,
o eine Zahl 0 oder 1 ist,
p, q und w unabhängig voneinander eine Zahl 1, 2, 3 oder 4 sind,
R⁴ gleich Wasserstoff, Hydroxy, Halogen, Amino oder Methyl ist,
oder eines ihrer Salze, ihrer Solvate oder der Solvate ihrer Salze.

2. Verbindung nach Anspruch 1, **dadurch gekennzeichnet, dass** sie der Formel 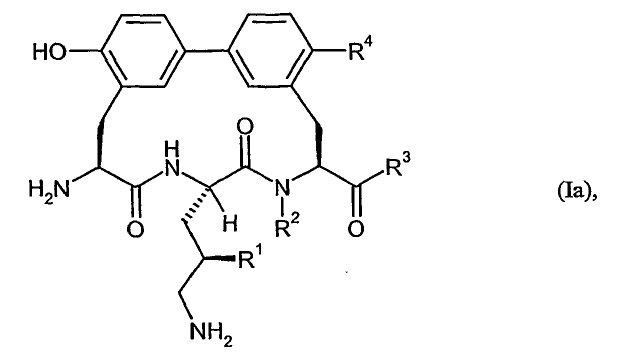 entspricht, in welcher
R¹ gleich Wasserstoff oder Hydroxy ist,
R² gleich Wasserstoff, Methyl oder Ethyl ist,
R³ wie oben definiert ist,
R⁴ gleich Wasserstoff, Hydroxy, Halogen, Amino oder Methyl ist,
oder eines ihrer Salze, ihrer Solvate oder der Solvate ihrer Salze.

3. Verbindung nach Anspruch 1 oder 2, **dadurch gekennzeichnet, dass**
R² gleich Wasserstoff ist.

4. Verbindung nach einem der Ansprüche 1 bis 3, **dadurch gekennzeichnet, dass**
R⁴ gleich Wasserstoff, Hydroxy, Chlor oder Methyl ist.

5. Verbindung nach einem der Ansprüche 1 bis 4, **dadurch gekennzeichnet, dass**
R⁵ gleich eine Gruppe der Formel 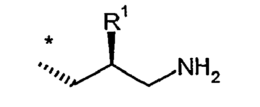 ist,
wobei
* die Anknüpfstelle an das Kohlenstoffatom ist,
R¹ gleich Wasserstoff oder Hydroxy ist,
R² gleich Wasserstoff ist,
R³ gleich eine Gruppe der Formel 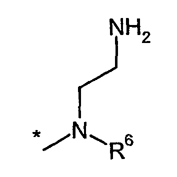 ist,
wobei
* die Anknüpfstelle an das Stickstoffatom ist,
R⁶ gleich eine Gruppe der Formel 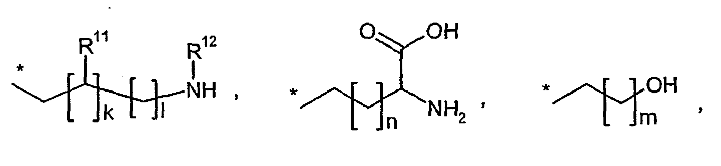 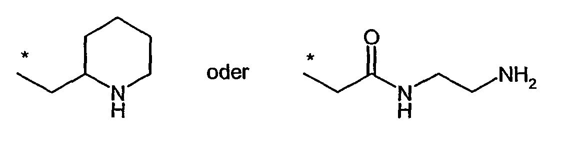 ist,
worin
* die Anknüpfstelle an das Stickstoffatom ist,
R¹¹ gleich Wasserstoff, Amino oder Hydroxy ist,
R¹² gleich Wasserstoff oder Methyl ist,
k eine Zahl 0 oder 1 ist,
l eine Zahl 1, 2, 3 oder 4 ist,
und
m und n unabhängig voneinander eine Zahl 1, 2 oder 3 sind,
R⁴ gleich Hydroxy ist,
oder eines ihrer Salze, ihrer Solvate oder der Solvate ihrer Salze.

6. Verbindung nach einem der Ansprüche 1 bis 4, **dadurch gekennzeichnet, dass**
R⁵ gleich eine Gruppe der Formel 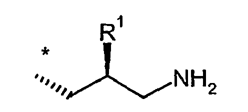 ist,
wobei
* die Anknüpfstelle an das Kohlenstoffatom ist,
R¹ gleich Wasserstoff oder Hydroxy ist,
R² gleich Wasserstoff ist,
R³ gleich eine Gruppe der Formel 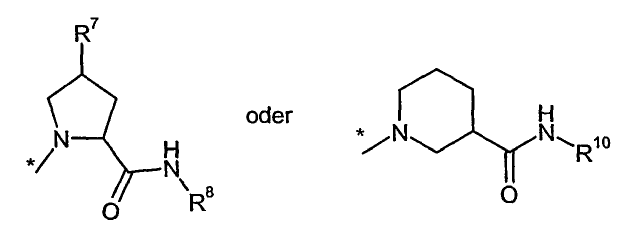 ist,
wobei
* die Anknüpfstelle an das Stickstoffatom ist,
R⁷ gleich Wasserstoff, Amino oder Hydroxy ist,
R⁸ und R¹⁰ unabhängig voneinander eine Gruppe der Formel 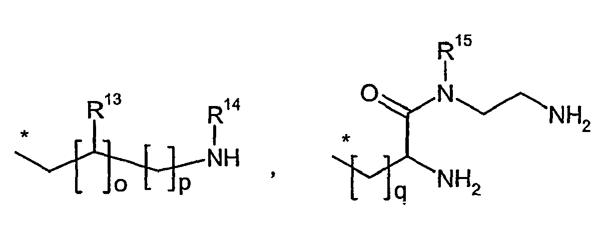 oder 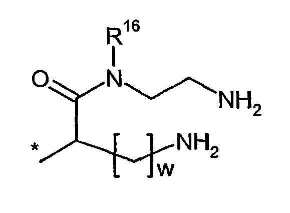 sind,
worin
* die Anknüpfstelle an das Stickstoffatom ist,
R¹³ gleich Wasserstoff, Amino oder Hydroxy ist,
R¹⁴ gleich Wasserstoff oder Methyl ist,
R¹⁵ und R¹⁶ unabhängig voneinander Wasserstoff, Aminoethyl oder Hydroxyethyl sind,
o eine Zahl 0 oder 1 ist,
p, q und w unabhängig voneinander eine Zahl 1, 2, 3 oder 4 sind,
R⁴ gleich Hydroxy ist,
oder eines ihrer Salze, ihrer Solvate oder der Solvate ihrer Salze.

7. Verbindung nach einem der Ansprüche 1 bis 4, **dadurch gekennzeichnet, dass**
R⁵ gleich eine Gruppe der Formel 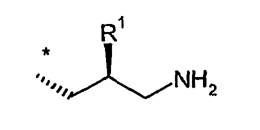 ist,
wobei
* die Anknüpfstelle an das Kohlenstoffatom ist,
R¹ gleich Wasserstoff oder Hydroxy ist,
R² gleich Wasserstoff ist,
R³ gleich eine Gruppe der Formel 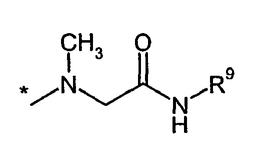 ist,
wobei
* die Anknüpfstelle an das Stickstoffatom ist,
R⁹ eine Gruppe der Formel 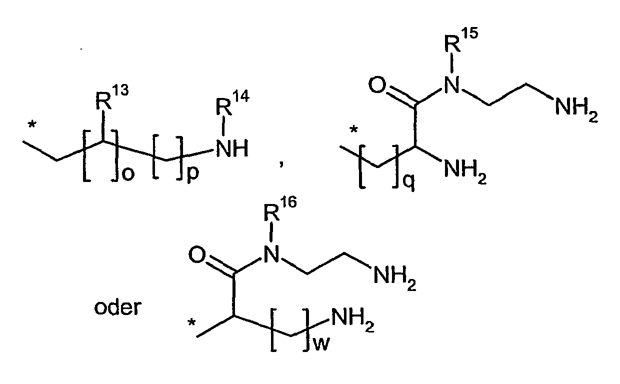 ist,
worin
* die Anknüpfstelle an das Stickstoffatom ist,
R¹³ gleich Wasserstoff, Amino oder Hydroxy ist,
R¹⁴ gleich Wasserstoff oder Methyl ist,
R¹⁵ und R¹⁶ unabhängig voneinander Wasserstoff, Aminoethyl oder Hydroxyethyl sind,
o eine Zahl 0 oder 1 ist,
p, q und w unabhängig voneinander eine Zahl 1, 2, 3 oder 4 sind,
R⁴ gleich Hydroxy ist,
oder eines ihrer Salze, ihrer Solvate oder der Solvate ihrer Salze.

8. Verfahren zur Herstellung einer Verbindung der Formel (I) nach Anspruch 1 oder eines ihrer Salze, Solvate oder der Solvate ihrer Salze, **dadurch gekennzeichnet, dass**
[A] eine Verbindung der Formel 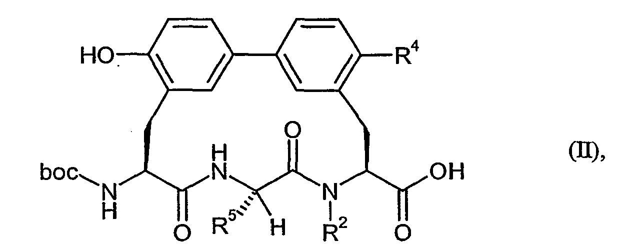 worin R², R⁴ und R⁵ die in Anspruch 1 angegebene Bedeutung haben und boc gleich *tert-*Butoxycarbonyl ist,
in einem zweistufigen Verfahren zunächst in Gegenwart von einem oder mehreren Dehydratisierungsreagenzien mit einer Verbindung der Formel
HR³ (III),
worin R³ die in Anspruch 1 angegebene Bedeutung hat,
und anschließend mit einer Säure und/oder durch Hydrogenolyse umgesetzt wird,
oder
[B] eine Verbindung der Formel 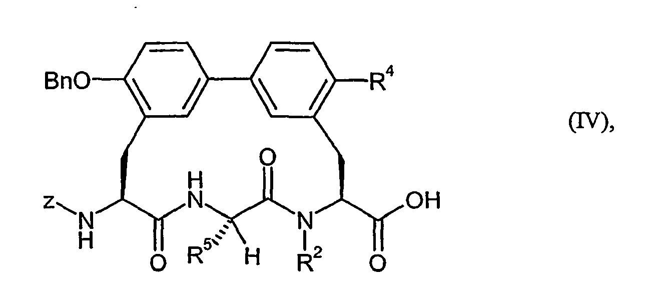 worin R², R⁴ und R⁵ die in Anspruch 1 angegebene Bedeutung haben und Z gleich Benzyloxycarbonyl ist,
in einem zweistufigen Verfahren zunächst in Gegenwart von einem oder mehreren Dehydratisierungsreagenzien mit einer Verbindung der Formel
HR³ (III),
worin R³ die in Anspruch 1 angegebene Bedeutung hat, und anschließend mit einer Säure oder durch Hydrogenolyse umgesetzt wird.

9. Verfahren zur Herstellung einer Verbindung der Formel (I) nach Anspruch 1 oder eines ihrer Solvate, **dadurch gekennzeichnet, dass** ein Salz der Verbindung oder ein Solvat eines Salzes der Verbindung durch Chromatographie unter Zusatz einer Base in die Verbindung überführt wird.

10. Verbindung nach einem der Ansprüche 1 bis 7 zur Behandlung und/oder Prophylaxe von Krankheiten.

11. Verwendung einer Verbindung nach einem der Ansprüche 1 bis 7 zur Herstellung eines Arzneimittels zur Behandlung und/oder Prophylaxe von Krankheiten.

12. Verwendung einer Verbindung nach einem der Ansprüche 1 bis 7 zur Herstellung eines Arzneimittels zur Behandlung und/oder Prophylaxe von bakteriellen Erkrankungen.

13. Arzneimittel enthaltend mindestens eine Verbindung nach einem der Ansprüche 1 bis 7 in Kombination mit mindestens einem inerten, nichttoxischen, pharmazeutisch geeigneten Hilfsstoff.

14. Arzneimittel nach Anspruch 13 zur Behandlung und/oder Prophylaxe von bakteriellen Infektionen.

15. Verwendung einer Verbindung nach einem der Ansprüche 1 bis 7 oder eines Arzneimittels nach Anspruch 13 oder 14 in einer antibakteriell wirksamen Menge zur Herstellung eines Arzneimittels zur Bekämpfung von bakteriellen Infektionen in Menschen und Tieren.

## Claims

1. Compound of formula 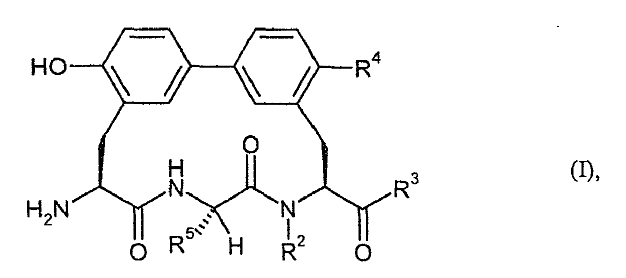 in which
R⁵ represents a group of formula 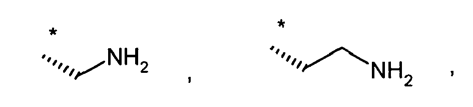 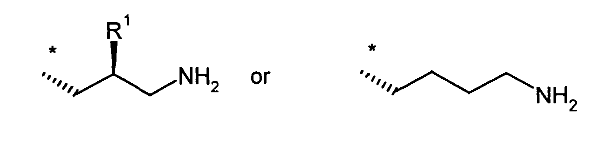 whereby
* is the linkage site to the carbon atom,
R¹ represents hydrogen or hydroxy,
R² represents hydrogen, methyl or ethyl,
R³ represents a group of formula 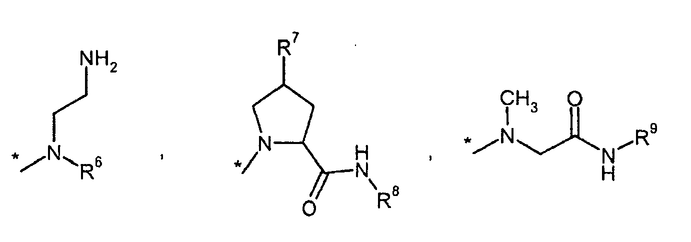 or 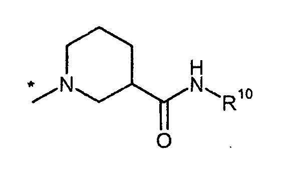 whereby
* is the linkage site to the nitrogen atom,
R⁶ represents a group of formula 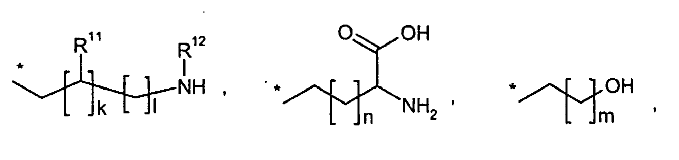 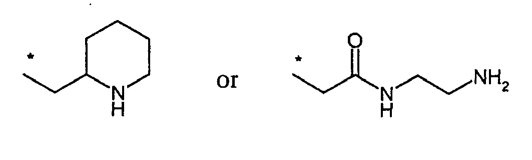 in which
* is the linkage site to the nitrogen atom,
R¹¹ represents hydrogen, amino or hydroxy,
R¹² represents hydrogen or methyl,
k is a number 0 or 1,
l is a number 1, 2, 3 or 4,
and
m and n independently of one another are a number 1, 2 or 3,
R⁷ represents hydrogen, amino or hydroxy,
R⁸, R⁹ and R¹⁰ independently of one another represent a group of formula 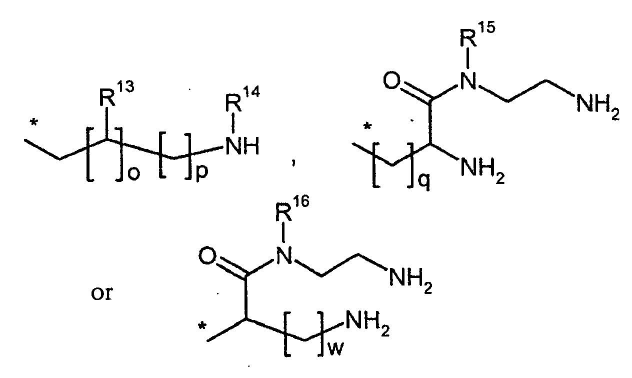 in which
* is the linkage site to the nitrogen atom
R¹³ represents hydrogen, amino or hydroxy,
R¹⁴ represents hydrogen or methyl,
R¹⁵ and R¹⁶ independently of one another represent hydrogen, aminoethyl or hydroxyethyl,
o is a number 0 or 1,
p, q and w independently of one another are a number 1, 2, 3 or 4,
R⁴ represents hydrogen, hydroxy, halogen, amino or methyl,
or one of its salts, its solvates or the solvates of its salts.

2. Compound according to claim 1, **characterized in that** it corresponds to formula 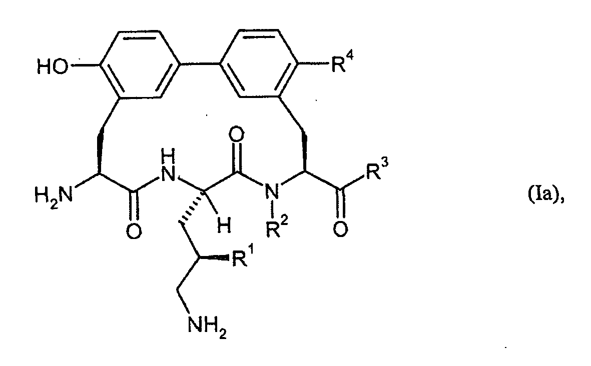 in which
R¹ represents hydrogen or hydroxy,
R² represents hydrogen, methyl or ethyl,
R³ is as defined above,
R⁴ represents hydrogen, hydroxy, halogen, amino or methyl,
or one of its salts, its solvates or the solvates of its salts.

3. Compound according to claim 1 or 2, **characterized in that**
R² represents hydrogen.

4. Compound according to any one of claims 1 to 3, **characterized in that**
R⁴ represents hydrogen, hydroxy, chlorine or methyl.

5. Compound according to any one of claims 1 to 4, **characterized in that**
R⁵ represents a group of formula 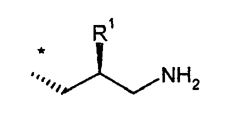 whereby
* is the linkage site to the carbon atom,
R¹ represents hydrogen or hydroxy,
R² represents hydrogen,
R³ represents a group of formula 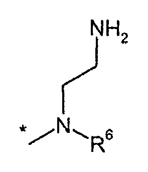 whereby
* is the linkage site to the nitrogen atom,
R⁶ represents a group of formula 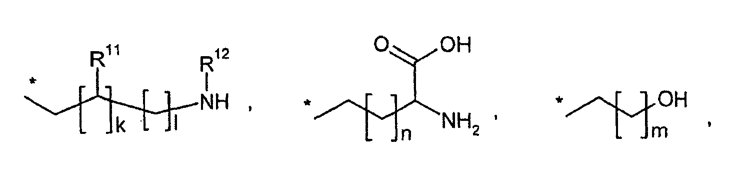 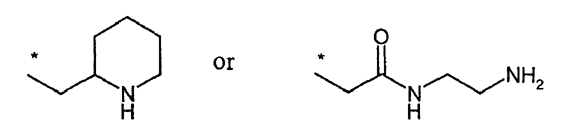 in which
* is the linkage site to the nitrogen atom,
R¹¹ represents hydrogen, amino or hydroxy,
R¹² represents hydrogen or methyl,
k is a number 0 or 1,
l is a number 1, 2, 3 or 4,
and
m and n independently of one another are a number 1, 2 or 3,
R⁴ represents hydroxy,
or one of its salts, its solvates or the solvates of its salts.

6. Compound according to any one of claims 1 to 4, **characterized in that**
R⁵ represents a group of formula 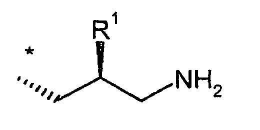 whereby
* is the linkage site to the carbon atom,
R¹ represents hydrogen or hydroxy,
R² represents hydrogen,
R³ represents a group of formula 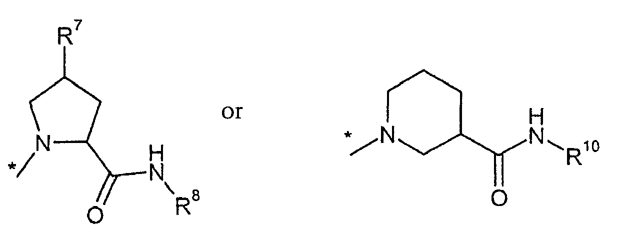 whereby
* is the linkage site to the nitrogen atom,
R⁷ represents hydrogen, amino or hydroxy,
R⁸ and R¹⁰ independently of one another represent a group of formula 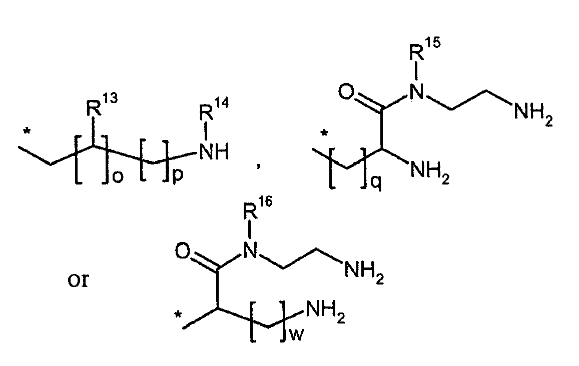 in which
* is the linkage site to the nitrogen atom,
R¹³ represents hydrogen, amino or hydroxy,
R¹⁴ represents hydrogen or methyl,
R¹⁵ and R¹⁶ independently of one another represent hydrogen, aminoethyl or hydroxyethyl,
o is a number 0 or 1,
p, q and w independently of one another are a number 1, 2, 3 or 4,
R⁴ represents hydroxy,
or one of its salts, its solvates or the solvates of its salts.

7. Compound according to any one of claims 1 to 4, **characterized in that**
R⁵ represents a group of formula 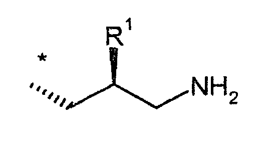 whereby
* is the linkage site to the carbon atom,
R¹ represents hydrogen or hydroxy,
R² represents hydrogen,
R³ represents a group of formula 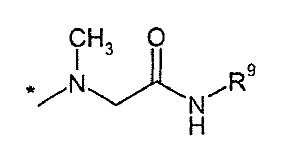 whereby
* is the linkage site to the nitrogen atom,
R⁹ represents a group of formula 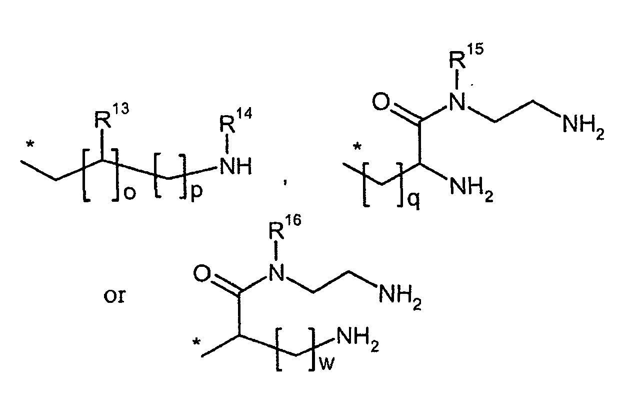 in which
* is the linkage site to the nitrogen atom,
R¹³ represents hydrogen, amino or hydroxy,
R¹⁴ represents hydrogen or methyl,
R¹⁵ and R¹⁶ independently of one another represent hydrogen, aminoethyl or hydroxyethyl,
o is a number 0 or 1,
p, q and w independently of one another are a number 1, 2, 3 or 4,
R⁴ represents hydroxy,
or one of its salts, its solvates or the solvates of its salts.

8. Method for preparing a compound of formula (I) according to claim 1 or one of its salts, solvates or solvates of its salts, **characterized in that**
[A] a compound of formula 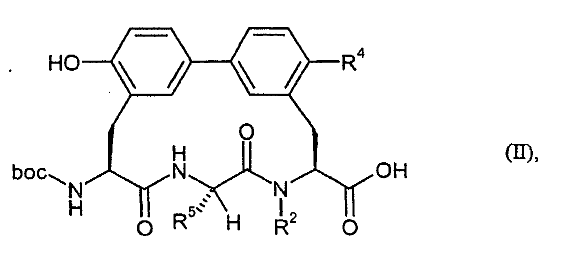 in which R², R⁴ and R⁵ have the meaning indicated in claim 1, and boc represents *tert*-butoxycarbonyl,
is reacted in a two-stage process firstly in the presence of one or more dehydrating reagents with a compound of formula
HR³ (III)
in which R³ has the meaning indicated in claim 1,
and subsequently with an acid and/or by hydrogenolysis,
or
[B] a compound of formula 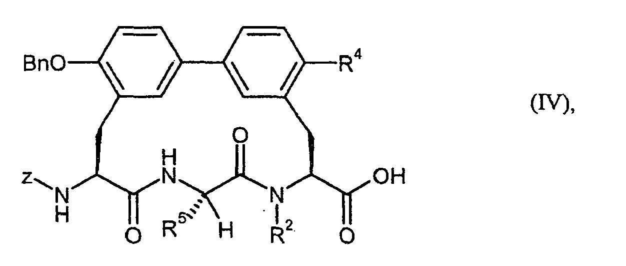
in which R², R⁴ and R⁵ have the meaning indicated in claim 1 and Z represents benzyloxycarbonyl,
is reacted in a two-stage process firstly in the presence of one or more dehydrating reagents with a compound of formula
HR³ (III)
in which R³ has the meaning indicated in claim 1,
and subsequently with an acid or by hydrogenolysis.

9. Method for preparing a compound of formula (I) according to claim 1 or one of its solvates, **characterized in that** a salt of the compound or a solvate of a salt of the compound is converted into the compound by chromatography with addition of a base.

10. Compound according to any one of claims 1 to 7 for the treatment and/or prophylaxis of diseases.

11. Use of a compound according to any one of claims 1 to 7 for the production of a medicament for the treatment and/or prophylaxis of diseases.

12. Use of a compound according to any one of claims 1 to 7 for the production of a medicament for the treatment and/or prophylaxis of bacterial diseases.

13. Medicament comprising at least one compound according to any one of claims 1 to 7 in combination with at least one inert, non-toxic, pharmaceutically acceptable excipient.

14. Medicament according to claim 13 for the treatment and/or prophylaxis of bacterial infections.

15. Use of a compound according to any one of claims 1 to 7 or of a medicament according to claim 13 or 14 in an antibacterially effective amount for the production of a medicament for controlling bacterial infections in humans and animals.

## Revendications

1. Composé de formule 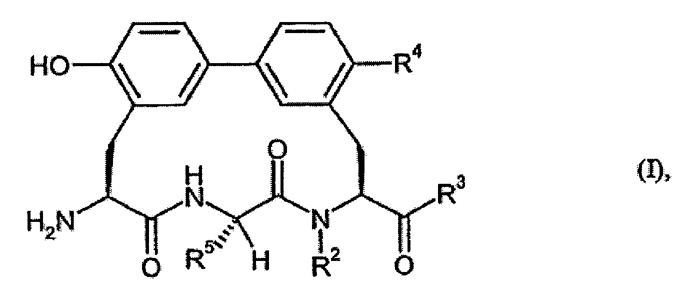 dans laquelle
R⁵ désigne un groupe de formule 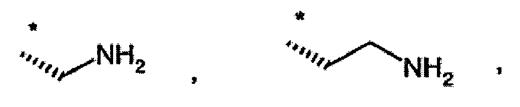 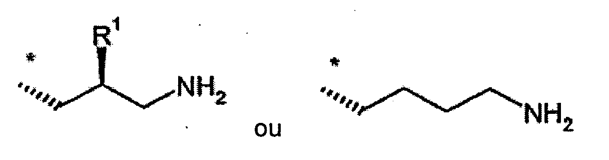 dans laquelle
* est le site de liaison à l'atome de carbone,
R¹ est un atome d'hydrogène ou un groupe hydroxy,
R² est un atome d'hydrogène, un groupe méthyle ou éthyle,
R³ est un groupe de formule 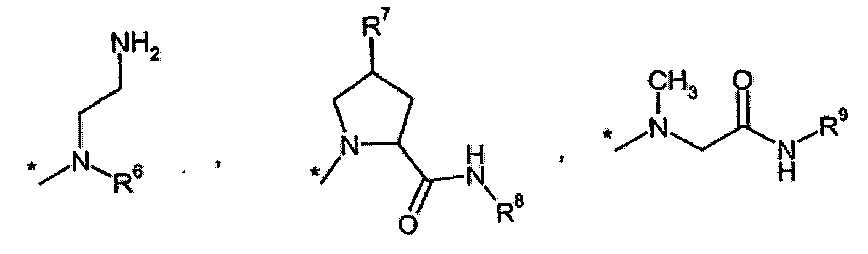 ou 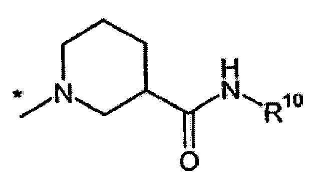 dans laquelle
* est le site de liaison à l'atome d'azote,
R⁶ est un groupe de formule 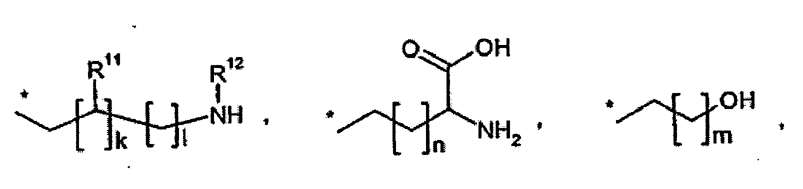 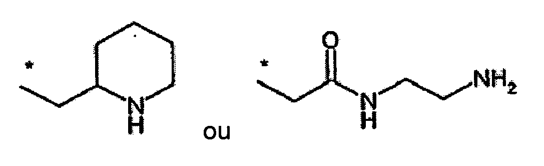 dans laquelle
* est le site de liaison à l'atome d'azote,
R¹¹ est un atome d'hydrogène, un groupe amino ou hydroxy,
R¹² est un atome d'hydrogène ou un groupe méthyle,
k est un nombre de 0 ou 1,
l est un nombre de 1, 2, 3 ou 4,
et
m et n indépendamment l'un de l'autre désignent un nombre de 1, 2 ou 3,
R⁷ est un atome d'hydrogène, un groupe amino ou hydroxy,
R⁸, R⁹ et R¹⁰ indépendamment les uns des autres, désignent un groupe de formule 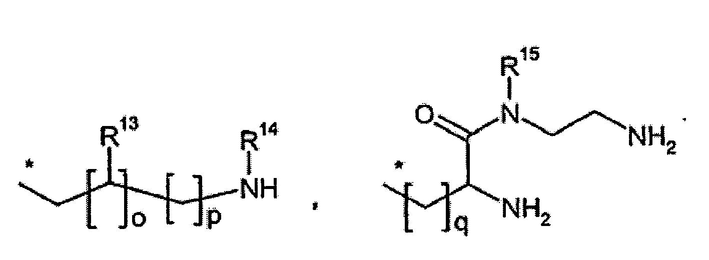 ou 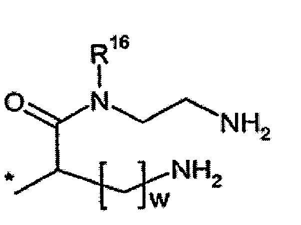 dans laquelle
* est le site de liaison à l'atome d'azote,
R¹³ est un atome d'hydrogène, un groupe amino ou hydroxy,
R¹⁴ est un atome d'hydrogène ou un groupe méthyle,
R¹⁵ et R¹⁶ sont indépendamment l'un de l'autre, un atome d'hydrogène, un groupe amino-éthyle ou hydroxyéthyle,
o est un nombre de 0 ou 1,
p, q et w sont indépendamment les uns des autres, un nombre de 1, 2, 3
ou 4,
R⁴ est un atome d'hydrogène, un groupe hydroxy, un atome d'halogène, un groupe amino ou méthyle,
ou l'un de ses sels, leurs solvates ou les solvates de leurs sels.

2. Composé selon la revendication 1, **caractérisé en ce qu'**il correspond à la formule 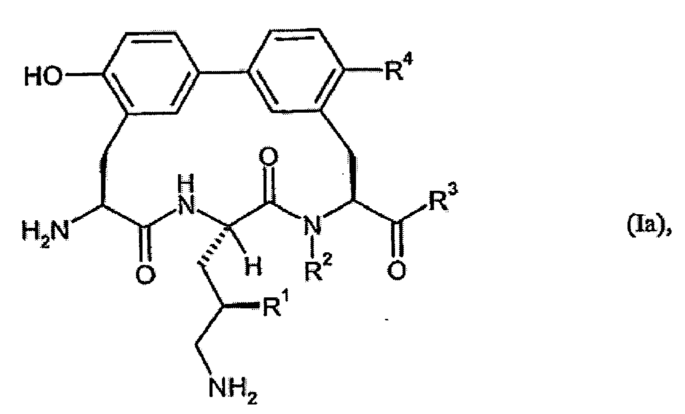 dans laquelle
R¹ est un atome d'hydrogène ou un groupe hydroxy,
R² est un atome d'hydrogène, un groupe méthyle ou éthyle,
R³ est tel que défini ci-dessus,
R⁴ est un atome d'hydrogène, un groupe hydroxy, un atome d'halogène, un groupe amino ou méthyle,
ou l'un de leurs sels, leurs solvates ou les solvates de leurs sels.

3. Composé selon la revendication 1 ou 2, **caractérisé en ce que**
R² est un atome d'hydrogène.

4. Composé selon l'une des revendications 1 à 3, **caractérisé en ce que**
R⁴ est un atome d'hydrogène, un groupe hydroxy, un atome de chlore ou un groupe méthyle.

5. Composé selon l'une des revendications 1 à 4, **caractérisé en ce que**
R⁵ est un groupe de formule 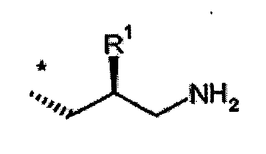 dans laquelle
* est le site de liaison à l'atome de carbone,
R¹ est un atome d'hydrogène ou un groupe hydroxy,
R² est un atome d'hydrogène,
R³ est un groupe de formule 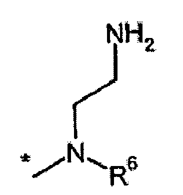 dans laquelle
* est le site de liaison à l'atome d'azote,
R⁶ est un groupe de formule 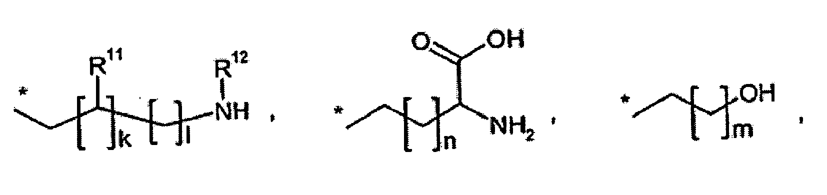 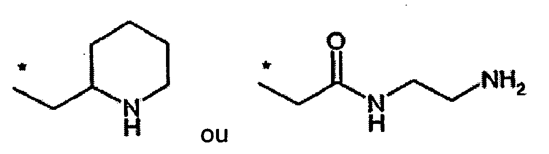 dans laquelle
* est le site de liaison à l'atome d'azote,
R¹¹ est un atome d'hydrogène, un groupe amino ou hydroxy,
R¹² est un atome d'hydrogène ou un groupe méthyle,
k est un nombre de 0 ou 1,
l est un nombre de 1, 2, 3 ou 4
et
m et n sont indépendamment l'un de l'autre, un nombre de 1, 2 ou 3,
R⁴ est un groupe hydroxy
ou l'un de leurs sels, leurs solvates ou les solvates de leurs sels.

6. Composé selon l'une des revendications 1 à 4, **caractérisé en ce que** R⁵ est un groupe de formule 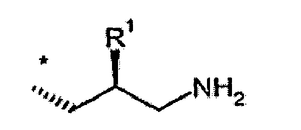 dans laquelle
* est le site de liaison à l'atome d'azote,
R¹ est un atome d'azote ou un groupe hydroxy,
R² est un atome d'hydrogène,
R³ est un groupe de formule 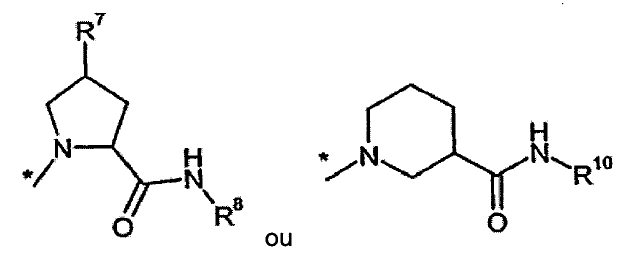 dans laquelle
* est le site de liaison à l'atome d'azote,
R⁷ est un atome d'hydrogène, un groupe amino ou hydroxy,
R⁸ et R¹⁰ sont indépendamment les uns des autres, un groupe de formule 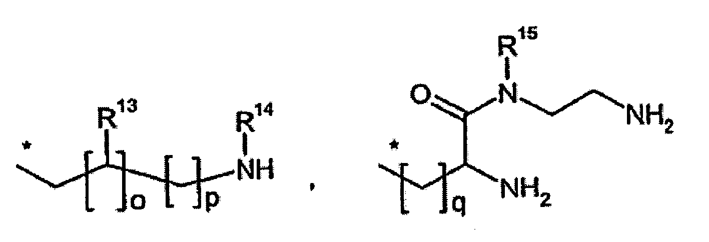 ou 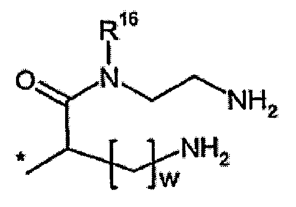 dans laquelle
* est le site de liaison à l'atome d'azote,
R¹³ est un atome d'hydrogène, un groupe amino ou hydroxy,
R¹⁴ est un atome d'hydrogène ou un groupe méthyle,
R¹⁵ et R¹⁶ sont indépendamment l'un de l'autre, un atome d'hydrogène, un groupe amino-éthyle ou hydroxy-éthyle,
o est un nombre de 0 ou 1,
p, q, et w sont indépendamment les uns des autres, un nombre de 1, 2, 3 ou 4,
R⁴ est un groupe hydroxy
ou l'un de leurs sels, leurs solvates ou les solvates de leurs sels.

7. Composé selon l'une des revendications 1 à 4, **caractérisé en ce que** R⁵ est un groupe de formule 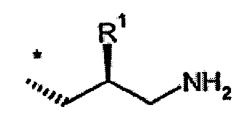 dans laquelle
* est le site de liaison à l'atome de carbone,
R¹ est un atome d'hydrogène ou un groupe hydroxy,
R² est un atome d'hydrogène,
R³ est un groupe de formule 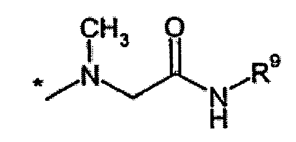 dans laquelle
* est le site de liaison à l'atome d'azote,
R⁹ est un groupe de formule 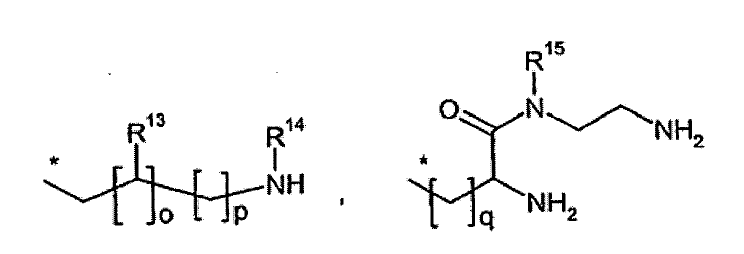 ou 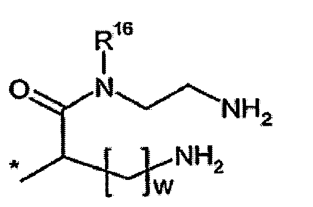 dans lequel
* est le site de liaison à l'atome d'azote,
R¹³ est un atome d'hydrogène, un groupe amino ou hydroxy,
R¹⁴ est un atome d'hydrogène ou un groupe méthyle,
R¹⁵ et R¹⁶ sont indépendamment l'un de l'autre, un atome d'hydrogène, un groupe amino-éthyle ou un groupe hydroxy-éthyle,
o est un nombre de 0 ou 1,
p, q et w sont indépendamment les uns des autres, un nombre de 1, 2, 3 ou 4,
R⁴ est un groupe hydroxy,
ou l'un de leurs sels, leurs solvates ou les solvates de leurs sels.

8. Procédé de production d'un composé de formule (I) selon la revendication 1 ou l'un de leurs sels, solvates ou solvates de leurs sels, **caractérisé en ce que**
[A] un composé de formule 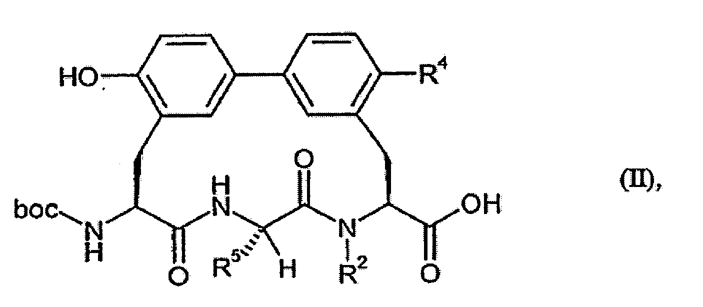 dans laquelle R², R⁴ et R⁵ qui ont la signification indiquée à la revendication 1 et boc désignant un groupe tert-butoxycarbonyel,
est converti dans un procédé en deux étapes, tout d'abord en présence d'un ou plusieurs réactifs de déshydratation avec un composé de formule
HR³ (III),
dans laquelle R³ a la signification indiquée à la revendication 1,
et ensuite avec un acide et/ou par hydrogénolyse,
ou
[B] un composé de formule 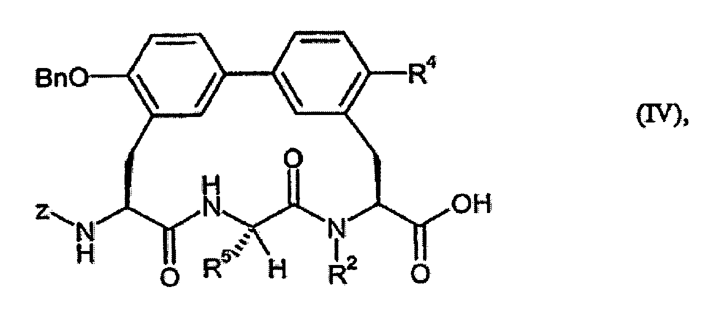
dans laquelle R², R⁴ et R⁵ ont la signification indiquée à la revendication 1 et Z est un groupe benzyloxycarbonyle,
est converti dans un procédé en deux étapes tout d'abord en présence d'un ou de plusieurs réactifs de déshydratation avec un composé de formule
HR³ (III),
dans laquelle R³ a la signification indiquée à la revendication 1
et ensuite, avec un acide ou par hydrogénolyse.

9. Procédé de production d'un composé de formule (1) selon la revendication 1 ou de l'un de ses solvates, **caractérisé en ce qu'**un sel du composé ou un solvate d'un sel du composé est converti en le composé par chromatographie en ajoutant une base.

10. Composé selon l'une des revendications 1 à 7, pour le traitement et/ou la prophylaxie de maladies.

11. Utilisation d'un composé selon l'une des revendications 1 à 7, pour la production d'un médicament pour le traitement et/ou la prophylaxie de maladies.

12. Utilisation d'un composé selon l'une des revendications 1 à 7, pour la production d'un médicament pour le traitement et/ou la prophylaxie de maladies bactériennes.

13. Médicament contenant au moins un composé selon l'une des revendications 1 à 7, en combinaison avec au moins un adjuvant inerte, non toxique, pharmaceutiquement approprié.

14. Médicament selon la revendication 13 pour le traitement et/ou la prophylaxie d'infections bactériennes.

15. Utilisation d'un composé selon l'une des revendications 1 à 7, ou d'un médicament selon la revendication 13 ou 14, en une quantité efficace d'un point de vue anti-bactérien, pour la production d'un médicament pour lutter contre les infections bactériennes chez l'être humain et l'animal.
